# EUROPÄISCHE PATENTSCHRIFT

(11) **EP 2 838 905 B1**
(45) Veröffentlichungstag und Bekanntmachung des Hinweises auf die Patenterteilung: **25.09.2019**
(21) Anmeldenummer: 13705994.5
(22) Anmeldetag: 25.02.2013
(51) Int. Cl.: C07F 7/18, C08G 77/20, C08G 77/388, C07F 7/08

(54) **NEUE, EINFACH SYNTHETISIERBARE, SPONTAN WASSERLÖSLICHE, IM WESENTLICHEN VOC FREIE, UMWELTFREUNDLICHE (METH)ACRYLAMIDO-FUNKTIONELLE SILOXANOLSYSTEME, VERFAHREN ZU IHRER HERSTELLUNG SOWIE VERWENDUNG**
NOVEL, MONO-SYNTHESIZABLE, SPONTANEOUSLY WATER-SOLUBLE, SUBSTANTIALLY VOC-FREE, ENVIRONMENTALLY FRIENDLY (METH)ACRYLAMIDO-FUNCTIONAL SILOXANOL SYSTEMS, PROCESS FOR THEIR PREPARATION AND USE
NOUVEAUX SYSTÈMES DE SILOXANOL À FONCTION (METH)ACRYLAMIDO, FACILEMENT SYNTHÉTISABLES, SPONTANÉMENT HYDROSOLUBLES, SENSIBLEMENT EXEMPTS DE COMPOSÉS ORGANIQUES VOLATILS (VOC), BIODÉGRADABLES, ET LEUR PROCÉDÉ DE PRODUCTION ET LEUR UTILISATION

(30) Priorität: 20.04.2012 DE 102012206510
(43) Veröffentlichungstag der Anmeldung: 25.02.2015
(73) Patentinhaber: Evonik Degussa GmbH, 45128 Essen (DE)
(72) Erfinder: WASSMER, Christian, 79688 Hausen (DE); STANDKE, Burkhard, 79540 Lörrach (DE); SCHLOSSER, Thomas, 79594 Inzlingen (DE); KRAUSE, Regina, 79618 Rheinfelden (DE)
(74) Vertreter: Evonik Patent Association
(86) Internationale Anmeldenummer: PCT/EP2013/053651
(87) Internationale Veröffentlichungsnummer: WO 2013/156185

(56) Entgegenhaltungen:
- EP-A1- 0 222 045
- EP-A1- 0 621 607
- EP-A1- 2 277 496
- EP-A2- 0 425 121
- CN-A- 101 798 464
- US-A- 3 900 679
- US-A- 4 927 951
- BOONLOM THAVORNYUTIKARN ET AL: "Synthesis and Characterization of UV-Curable Poly(dimethylsiloxane) Dimethacrylate", MACROMOLECULAR SYMPOSIA, Bd. 264, Nr. 1, 1. April 2008 (2008-04-01) , Seiten 144-148, XP0055064752, ISSN: 1022-1360, DOI: 10.1002/masy.200850423

## Beschreibung

Die Erfindung betrifft eine Zusammensetzung und ein Verfahren zur Herstellung der Zusammensetzung umfassend (Meth)acrylamido-funktionelle Siloxanole, vorzugsweise im Wesentlichen wasserlösliche (Meth)acrylamido-funktionelle Siloxanole, sowie deren Verwendung.

Für den Einsatz von Glasfasern in Faserverbundwerkstoffen wird die Glasfaser häufig mit funktionalisierten Silanen oberflächenbehandelt. Dies geschieht gängigerweise mit Hilfe wässriger Schlichten in denen das organofunktionelle Silan gelöst wird. Abhängig von der chemischen Funktion der Silane können die gewünschten Eigenschaften wie zum Beispiel Faserstärke oder auch Schneidbarkeit (speziell für die Kurzfaserverstärkung) positiv beeinflusst werden. Die organofunktionellen Silane tragen dabei auch wesentlich zur Haftungsvermittlung zwischen der anorganischen Faser und dem organischen Harz bei. Obwohl die Applikation mittels wässriger Schlichten angestrebt wird, erfolgt die Herstellung der organofunktionellen Silane noch in organischen Lösemitteln.

So werden beispielsweise spezielle Methacryl-funktionalisierte Silane wie zum Beispiel 3-Methacryloxypropyl-trimethoxysilan in Faserverbundwerkstoffen eingesetzt, beispielhaft erwähnt seien Thermo- und Duroplaste, um die Performance des Faserverbundwerkstoffs zu erhöhen. Auch in anderen Anwendungen, wie bei der Füllstoffmodifizierung, in Beschichtungen oder in Kleb-/Dichtstoffen werden diese funktionalisierten Silane als Haftvermittler zwischen organischer und anorganischer Matrix eingesetzt.

Eine weitere Anwendung liegt in der Modifizierung von speziellen Eigenschaften wie beispielsweise der Erhöhung der Schneidbarkeit von Glasfasern. Einige der dafür genutzten Verbindungen sind Methacrylamidoalkylalkoxysilane, wie (RO)ₓRSiNH(CO)C(CH₃)=CH₂ oder auch Chrom (III) Methacrylsäure Chlor Komplexe wie zum Beispiel Volan® der Fa. Du Pont (R = C1 - C6 Alkylgruppe).

Um die Verbindungen in wässrigen Schlichten anzubieten, müssen sie eine gute Wasserlöslichkeit aufweisen. Die Chrom basierten Methacrylatverbindungen zeigen eine gute Wasserlöslichkeit. Jedoch haben sie den Nachteil schwermetallhaltig zu sein. Das Methacrylamidoalkylalkoxysilan führt in wässrigem Medium zur Hydrolyse der Alkoxygruppen und zur Freisetzung der korrespondierenden Alkohole Methanol (giftig) und Ethanol und damit zur Bildung von VOC (volatile organic compounds).

In WO 00/75148 A1 (hier Vergleichsbeispiel 1) wird eine Synthese ausgehend von Aminopropyltriethoxysilan mit einem Methacrylsäuremethylester in Gegenwart von Dibutylzinnoxid (DBTO) beschrieben. Diese Umsetzung weist eine Reihe von Nachteilen auf, zum einen wird für eine möglichst vollständige Umsetzung ein Überschuss an Methacrylsäureester von 100 % eingesetzt, der wieder abdestilliert werden muss. Damit ist die Raum-Zeit-Ausbeute schlecht. Zudem wird die Umsetzung bei hohen Temperaturen bei 165 - 170 °C durchgeführt, wodurch es zu Problemen aufgrund der Neigung zur Polymerisation der Acrylsäure kommt. Zur Vermeidung der Polymerisation muss ein Stabilisator eingesetzt werden. Als Katalysatoren für eine möglichst vollständige Umsetzung werden giftige, umweltgefährdende Zinn-organische Verbindungen wie zum Beispiel Di-butylzinnoxid (DBTO) eingesetzt. Ein weiterer Nachteil dieses Verfahrens ist die aufwändige Rektifikation des Reaktionsproduktes bei hohen Sumpftemperaturen und möglichst niedrigem Absolutdruck. Dafür ist ein weiterer Gasphasenstabilisator einzusetzen, um eine Polymerisation in der Kolonne zu vermeiden. Im Sumpf bleibt ein schwermetallhaltiger Rückstand zurück, der gesondert entsorgt werden muss. Das Destillationsprodukt, das kommerziell erhältliche Produkt Y-5997 von der Fa. Momentive (CH₃O)ₓ(C₂H₅O)₃₋ₓSi(CH₂)₃NH(CO)C(CH₃)=CH₂, ist in Wasser nahezu unlöslich.

US3,900,679 offenbart Glasfaserschlichten sowie Zusammensetzungen zur Herstellung von glasfaserverstärkten Materialien basierend auf Umsetzungsprodukten von Acrylsäue mit Aminopropyltriemthoxysilan ohne Gegenwart von Wasser. US4,927,951 betrifft die wasserfreie Umsetzung von Methacryloylchlorid und einem Aminosilan in Benzol. EP0621607 offenbart die Verwendung eines monomeren Acrylamidotrimethoxysilans zur Beschichtung von Kabeln zur Verbesserung der dielektrischen Eigenschaften. EP0222045 offenbart die Verwendung von Acrylamidotriethoxysilanen in bildgebenden Materialien. EP0425121A2 offenbart Silicon enthaltende Primer-Zusammensetzungen, die u.a. Acrylamidotrimethoxysilan aufweisen können. CN101798464A offenbart die Umsetzung von terminalen Amino-funktionellen Polydimethylsiliconölen mit Carbonsäureanhydriden. Boonlom Thavornyutikarn et al (Synthesis and Characterisation of UV-Curable Poly(dimethylsiloxane) Dimethacrylates, Bd. 264, Nr. 1, 1. April 2008 (2008-04-01), Seite 144-148) offenbart terminale Acrylamido-funktionelle Polydimethylsiloxane. EP2277496A1 offenbart die wasserfreie Herstellung von 3-(Methacrylamido)propyltriemthoxysilan durch Umsetzung von Methacryloylchlorid in Dichlormethan.ln US Patent 3,249,461 wird die Synthese von Methacrylamidopropylmethoxysilan durch die Umsetzung von Methacryloylchlorid in inerten wasserfreien Lösungsmitteln mit Aminoproyltrimethoxysilan beschrieben. Nachteilig in diesem Prozess ist die Freisetzung einer äquimolaren Menge an Chlorwasserstoff, der aus dem Prozess aufwändig abgetrennt werden muss. Außerdem verringert der Lösungsmittelanteil die Raum-Zeit-Ausbeute. Auch die Verwendung von Dinitrobenzol als Stabilisator ist von Nachteil.

Aufgabe der vorliegenden Erfindung war die Bereitstellung von (Meth)acrylamido-funktionellen, siliciumorganischen Verbindungen, die eine hervorragende Wasserlöslichkeit aufweisen sollen, insbesondere sollten sie spontan in Wasser löslich sein. Ferner sollten sie besonders umweltfreundlich, insbesondere ohne schwermetallhaltige Katalysatoren und/oder bedenkliche organische Lösemittel herstellbar sein. Gleichfalls sollte der Einsatz an Stabilisatoren, wie er im Stand der Technik notwendig ist, reduziert werden, insbesondere sollte ein Verfahren entwickelt werden, das ohne die Verwendung von Gasphasenstabilisatoren auskommt. Eine weitere Aufgabe bestand darin, ein Verfahren aufzufinden, das eine Herstellung als Eintopf-Reaktion erlaubt. Zudem bestand eine Aufgabe darin das Anwendungsspektrum der bereitzustellenden (Meth)acrylamido-funktionellen, siliciumorganischen Verbindungen aufzufächern und weiteren vorteilhaften Anwendungen zugänglich zu machen. Eine weitere Aufgabe bestand darin die Belastung an organischen Lösemitteln, insbesondere der reinen Kohlenwasserstoffe zu reduzieren, besser zu vermeiden und vorzugsweise auch die VOC Freisetzung bei der Anwendung durch Hydrolyse der Alkoxyfunktionen stark zu reduzieren. Zudem sollte die Anwendung beim Nutzer deutlich vereinfacht werden, indem die Zusammensetzung ggf. nach einer Verdünnung sofort appliziert werden kann und vorzugsweise selbst in wässriger Lösung ggf. nach einer Verdünnung lagerstabil ist. Daher bestand eine weitere Aufgabe darin, dass der Anwender anders als bei Systemen des Standes der Technik keine langen Vorlaufzeiten unter Verwendung mehrerer Komponenten, d.h. Zugabe von Wasser und Säure oder dergleichen, vor der Benutzung der (Meth)acrylamido-funktionellen siliciumorganischen Verbindungen beachten muss, sondern sie sofort ggf. nach spontaner Verdünnung in Wasser sofort verwenden kann.

Gelöst wurden die Aufgaben mit einer Zusammensetzung nach Anspruch 1 und 3 sowie mit dem Verfahren nach Anspruch 4 und der Verwendung nach Anspruch 18, wobei bevorzugte Ausführungsformen in den Unteransprüchen und in der Beschreibung erläutert sind. Ebenso wurde die Aufgabe gelöst durch eine gezielte wässrige Umsetzung von Aminosilanen, insbesondere von Aminoalkylalkoxysilanen, bevorzugt von Di- und/oder Triaminoalkyl-funktionellen Silanen, in Gegenwart von Feuchte oder wässrigen Medien (synonym zu: in Gegenwart von Wasser), besonders bevorzugt durch Hydrolyse und vorzugsweise Kondensation von N-(2-Aminoethyl)-3-aminopropyltrialkoxysilan und/oder 3-Aminopropyltrialkoxysilan zu Oligomeren, nachfolgend auch kurz Siloxanole genannt, und Umsetzung mit einem Acrylsäureanhydrid, insbesondere (Meth)acrylsäureanhydrid in wässrigem Medium. Die erhaltenen wasserlöslichen Acrylamido-funktionellen Siloxanole werden vorzugsweise zumindest teilweise, besonders bevorzugt vollständig hydrolysiert und optional im Wesentlichen (hydrolyse)alkoholfrei eingestellt. Ein großer Vorteil der Erfindung ist es, dass die so erhaltenen Acrylamido-funktionellen Siloxanole vorzugsweise ohne weitere Aufreinigung als Sumpfprodukt verwendet werden können. Damit können mit dem erfindungsgemäßen Verfahren und den erfindungsgemäßen Zusammensetzungen enthaltend Acrylamido-funktionellen Siloxanole besonders wirtschaftliche und ökologische Produkte angeboten werden.

Gegenstand der Erfindung ist eine Zusammensetzung umfassend Acrylamido-funktionelle Siloxanole, insbesondere im Wesentlichen wasserlösliche Acrylamido-funktionelle Siloxanole, die abgeleitet sind aus einer
a) Umsetzung einer Komponente A, die eine Aminoalkyl-funktionalisierte Siliciumverbindung ist, ausgewählt aus
   (i) einem Aminoalkyl-funktionellen Alkoxysilan der Formel I oder einem Gemisch von Aminoalkyl-funktionellen Alkoxysilanen der Formel I, jeweils in Gegenwart einer definierten Menge Wasser umgesetzt wird, oder
   (ii) einem Hydrolyse- oder Kondensationsprodukt mindestens eines Aminoalkyl-funktionellen Alkoxysilans der Formel I oder
   (iii) einem Gemisch, umfassend mindestens ein Aminoalkyl-funktionelles Alkoxysilan der Formel I und einem Hydrolyse- und/oder Kondensationsprodukt mindestens eines Aminoalkyl-funktionellen Alkoxysilans der Formel I,
   und nachfolgend mit einer Komponente B, die ein Acrylsäureanhydrid ist, umgesetzt wird und optional
b) Entfernen zumindest eines Teils des Hydrolysealkohols, und optional zumindest eines Teils des Wassers, das in (i) und optional in (ii) oder (iii) eingesetzt wurde, wobei optional zum Entfernen des Hydrolysealkohols in diesem Schritt weiteres Wasser zugesetzt wird, vorzugsweise wurden auch (ii) und (iii) durch Reaktion mit einer definierten Menge Wasser hergestellt.

Es wurde festgestellt, dass bei der direkten Umsetzung von Aminosilanen mit Acrylsäureanhydrid die unerwünschten Umesterungsprodukte auftreten, denn die freigesetzte Methacrylsäure reagiert unter Umesterung mit den Alkoxygruppen des Aminosilans.

Den Erfindern ist es überraschend gelungen, diese unerwünschten Umesterungsreaktionen, die bei einer Umsetzung von Aminosilanen mit (Meth)acrylsäureanhydrid auftreten, zu vermeiden. Die unerwünschte Umesterung kann vermieden werden, wenn vor der Umsetzung mit (Meth)acrylsäureanhydrid die Aminosilane durch Hydrolyse und optional Kondensation zu Siloxanen, vorzugsweise zu Siloxanolen oligomerisiert werden, wobei überraschend nachfolgende Acrylamidbildung zwischen den Aminoalkyl-funktionellen Siliciumverbindungen und dem (Meth)acrylsäureanhydrid gelingt.

Bevorzugt wird zur Hydrolyse der Aminoalkyl-funktionellen Silane eine definierte Menge Wasser eingesetzt, die vorzugsweise zwischen größer gleich 0,1 mol bis 4,5 mol, insbesondere zwischen 0,1 bis 2,0 mol Wasser/mol Silicium-Atome einschließlich der Grenzwerte liegt, bevorzugt zwischen größer gleich 0,3 mol bis 1,5 mol Wasser/mol Silicium-Atome der Aminoalkyl-funktionellen Siliciumverbindungen, besonders bevorzugt ist eine Wassermenge zwischen größer gleich 0,5 bis 1,0 mol Wasser/mol Silicium-Atome.

Die Sumpftemperatur kann bei der Umsetzung mit (Meth)acrylsäureanhydrid durch die ZutropfGeschwindigkeit von (Meth)acrylsäureanhydrid gesteuert werden. Durch eine Kühlung des Reaktionskolbens kann eine schnellere Zugabe von (Meth)acrylsäure erreicht werden. Die maximal mögliche Sumpftemperatur ist abhängig von dem Stabilisatorsystem in der Reaktionsmischung und dem Siedepunkt der eingesetzten Komponenten.

Als im Wesentlichen wasserlöslich gelten Acrylamido-funktionelle Siloxanole, die als solche wasserlöslich sind, insbesondere sich zu mindestens 3 bis 99,9 Gew.-% in Wasser lösen lassen oder sich entsprechend mit Wasser mischen lassen. Bevorzugte Wirkstoffkonzentrationen in Wasser liegen zwischen größer gleich 3 bis 50 Gew.-% in der Gesamtzusammensetzung, bevorzugt zwischen größer gleich 3 bis 40 Gew.-%, besonders bevorzugte alternative Konzentrationsbereiche liegen zwischen größer gleich 3 bis 10 Gew.-% oder auch zwischen 15 bis 45 Gew.-%. Dabei lassen sich die Acrylamido-funktionellen Siloxanole spontan in Wasser auflösen und bilden vorzugsweise klare Lösungen. Diese Lösungen sind lagerstabil, beispielsweise über mindestens drei Monate im geschlossenen Behälter bei 50 °C. Entsprechend einer Alternative gelten aber auch Acrylamido-funktionelle Siloxanole als wasserlöslich, die sich durch Zugabe von Säuren, Basen oder Puffern in wässriger Phase in Lösung bringen lassen und vorzugsweise klare Lösungen bilden.

Besonders bevorzugte Zusammensetzungen umfassen Acrylamido-funktionelle Siloxanole, insbesondere im Wesentlichen wasserlösliche Acrylamido-funktionelle Siloxanole, die abgeleitet sind aus einer Umsetzung a) einer Komponente A, einer Aminoalkyl-funktionalisierten Siliciumverbindung, die ausgewählt ist aus (ii) einem Hydrolyse- und/oder Kondensationsprodukt mindestens eines Aminoalkyl-funktionellen Alkoxysilans oder (iii) einem Gemisch, umfassend mindestens ein Aminoalkyl-funktionelles Alkoxysilan und ein Hydrolyse- und/oder Kondensationsprodukt mindestens eines Aminoalkyl-funktionellen Alkoxysilans, mit einer Komponente B, die ein Acrylsäureanhydrid ist, insbesondere der Formel IV, und optional b) Entfernen zumindest eines Teil des Hydrolysealkohols, und optional zumindest eines Teils des Wassers, wobei optional zum Entfernen des Hydrolysealkohols in diesem Schritt weiteres Wasser zugesetzt wird. Vorzugsweise werden die Acrylamido-funktionellen Siloxanole so lange mit Wasser versetzt und der Hydrolysealkohol entfernt bis quasi vollständige Hydrolyse der Alkoxygruppen eingetreten ist.

Erfindungsgemäß können grundsätzlich alle Aminoalkoxysilane zur Herstellung der Hydrolysate und Kondensate und nachfolgender Umsetzung mit (Methyl)acrylsäureanhydrid eingesetzt werden. Für die angestrebte verbesserte Löslichkeit werden bevorzugt Aminosilane mit einem primären und vorzugsweise mindestens einer sekundären Aminogruppe gewählt; diese mindestens Diamino-funktionellen Silane führen zu einer nochmals verbesserten Löslichkeit des entsprechenden (Meth)acrylamidoalkyl Siloxanols. Vorteil der zusätzlichen sekundären Aminogruppe ist, dass sie die bei der Reaktion freigesetzte (Meth)acrylsäure unter Bildung eines entsprechenden Salzes (Aminohydro(meth)acrylat) neutralisiert.

Das Aminohydro-(meth)acrylat kann basisch gespalten werden. Als Basen eigenen sich vorzugsweise basische Alkalisalze wie NaOH oder KOH, bevorzugt Alkalialkoxide wie NaOR oder KOR, bevorzugt für R = Alkyl- vorzugsweise Methyl- und besonders bevorzugt ist Kaliummethylat.

Wobei die Aminoalkyl-funktionelle Alkoxysilane der Formel I entsprechen

(R¹O)_{3-a-b}(R²)ₐSi(B)_{1+b} (I),

wobei die Gruppe-B in Formel I unabhängig einer Gruppe der Formel II

-(CH₂)_{c}-[(NH)(CH₂)_{d}]ₑ[(NH)](CH₂)_{f}]_{g}NH₍₂₋ₕ₎ R³ₕ (II)

entspricht, in Formel I mit R¹ unabhängig eine lineare, verzweigte oder cyclische Alkyl-Gruppe mit 1 bis 8 C-Atomen, insbesondere mit 1, 2, 3 oder 4 C-Atomen, bevorzugt Methyl, Ethyl oder Propyl, und R² unabhängig eine lineare, verzweigte oder cyclische Alkyl-Gruppe mit 1 bis 8 C-Atomen ist, insbesondere Methyl, Ethyl, Propyl, n-Butyl, iso-Butyl, tert.-Butyl, Pentyl, Hexyl, Heptyl oder Octyl, und in Formel II mit R³ unabhängig eine lineare, verzweigte oder cyclische Alkyl-, Aryl- oder Alkylaryl-Gruppe mit 1 bis 8 C-Atomen in Formel II, insbesondere Methyl, Ethyl, Butyl oder Benzyl, wobei h gleich 0 besonders bevorzugt ist, und in Formel I ist a unabhängig gleich 0 oder 1, b unabhängig gleich 0 oder 1, bevorzugt ist b gleich 0, und in Formel II ist c unabhängig ausgewählt aus 1, 2, 3, 4, 5 und 6, d ist unabhängig ausgewählt aus 1, 2, 3, 4, 5 und 6, e ist unabhängig ausgewählt aus 0, 1, 2, 3, 4, 5 und 6, f ist unabhängig ausgewählt aus 1, 2, 3, 4, 5 und 6, g ist unabhängig ausgewählt aus 0, 1, 2, 3, 4, 5 und 6, und h ist unabhängig 0 oder 1, alternativ bevorzugt sind e = g = 0 oder 1, und d = f = 2 oder 3 und h = 0 mit c = 3 und b = 0 und a = 0, besonders bevorzugte Kombinationen sind mit R¹ gleich Methyl oder Ethyl a = 0 und b = 0 mit c = 3 und g, e und h jeweils gleich 0; alternativ gleichfalls bevorzugt sind a = 0, b = 0, c = 3, e = 1, d = 1, 2 oder 3, vorzugsweise ist d = 2, und g = 0, h = 0, für Diamino-funktionelle Silane,
oder die Gruppe B entspricht der Formel III

-(CH₂)ⱼ-NH₂₋ₚ(CH₂-CH₂-NH₂)ₚ (III)

mit j = 1, 2 oder 3 und p = 0, 1 oder 2, bevorzugt ist p ausgewählt aus 1 und 2, zweckmäßig kann auch p = 0 sein.

Generell ist es bevorzugt, wenn das Aminoalkyl-funktionelle Alkoxysilan einem Diaminoalkylfunktionellen oder einem Triaminoalkyl-funktionellen Silan, vorzugsweise einem Diaminoalkylfunktionellen oder einem Triaminoalkyl-funktionellen Alkoxysilan der Formel I entspricht. Ebenso besonders bevorzugt sind Gemische der vorgenannten Silane, wie Aminosilan mit Diaminosilan oder auch Aminosilan mit Triaminosilan oder Diaminosilan mit Triaminosilan sowie auch Gemische mit drei oder mehr verschiedenen Aminosilanen der Formel I.

Als Acrylsäureanhydrid werden vorzugsweise Methacrylsäure oder (unsubstituiertes) Acrylsäureanhydrid eingesetzt, besonders bevorzugt gemäß der Formel IV

(CHR⁵=CR⁴CO)₂O (IV),

wobei R⁴ unabhängig ein Wasserstoffatom oder eine Methyl-Gruppe und R⁵ unabhängig ein Wasserstoffatom oder eine Methyl-Gruppe ist, vorzugsweise ist R⁵ ein Wasserstoffatom. Bevorzugt sind (CH₂=C(CH₃)CO)₂O und (CH₂=CHCO)₂O.

Die erfindungsgemäße Zusammensetzung, die aus der Umsetzung von (i), (ii) und/oder (iii) erhalten werden können, können idealisiert gemäß der nachfolgenden allgemeinen idealisierten Formel V für mindestens ein im Wesentlichen wasserlösliches Acrylamido-funktionelles Siloxanol dargestellt werden, wobei die Acrylamido-funktionellen Siloxanole vorzugsweise lineare, cyclische und vernetze Strukturen aufweisen können,

(R¹O)[(R¹O)₁₋ₐ(R²)ₐSi(C)_{1+b}O]ᵤ[(Y)Si(C)_{1+b}O]_{u'} R¹•(HX)_{z} (V),

wobei in der allgemeinen Formel V
- C eine Acrylamido-Gruppe ist und
- Y entspricht OR¹ oder in vernetzten und/oder raumvernetzten Strukturen unabhängig voneinander OR¹ oder O_{1/2},
- wobei R¹ Wasserstoff entspricht oder optional teilweise auch R¹ unabhängig eine lineare, verzweigte oder cyclische Alkyl-Gruppe mit 1 bis 8 C-Atomen ist, vorzugsweise ist R¹ zu kleiner 10 Mol.-%, bevorzugt kleiner 5 Mol.-% ein Alkyl, besonders bevorzugt kleiner 2 Mol.-%, vorzugsweise kleiner gleich 1 Mol.-%, und R² einer linearen, verzweigten oder cyclischen Alkyl-Gruppe mit 1 bis 8 C-Atomen entspricht, insbesondere gemäß der Definition von Formel (I),
- HX eine Säure darstellt, wobei X ein anorganischer oder organischer Säure-Rest ist,
- mit jeweils unabhängig a gleich 0 oder 1, jeweils unabhängig b gleich 0, 1 oder unabhängig zusätzlich optional 2, vorzugsweise ist b gleich 0, mit jeweils unabhängig eine ganze Zahl u größer gleich 2, u' größer gleich 0 und z größer gleich 0 und (u + u') ≥ 2, insbesondere ist z gleich 0 oder größer gleich 1, vorzugsweise kann z kleiner bis gleich der Anzahl an sekundären Stickstoffatomen des eingesetzten Aminosilans sein, gleichfalls bevorzugt kann z größer der Anzahl der sekundären Stickstoffatome sein,
- wobei die Zusammensetzung frei von Verdünnungsmittel, insbesondere organischen Lösemitteln, besonders bevorzugt von protischen organischen Lösemitteln, ist und beim Vernetzen keinen Alkohol mehr freisetzt.

Bevorzugt ist u im Mittel ausgewählt aus einer ganzen Zahl von 2 bis 500, insbesondere von 2 bis 150, vorzugsweise aus 2 bis 80, einschließlich aller dazwischen liegenden Zahlenwerte wie 2, 3, 4, 5, 6,7, 8, 9, 10, 15, 20, 25, 30, 35, 40, 45, 50, 55, 60, 65, 70, 75 und 80 sowie jeweils mit einer Schwankungsbreite von bis zu plus/minus 5, bevorzugt liegt u zwischen größer gleich 20 bis 80, besonders bevorzugt zwischen 20 bis 60, vorzugsweise zwischen größer gleich 20 bis 40. Wobei unabhängig davon u' im Mittel ausgewählt sein kann aus einer ganzen Zahl zwischen 0 bis 200, insbesondere von 0 bis 100, vorzugsweise aus 0, 1, 2, 3, 4, 5, 6, 7, 8, 9, 10, 15, 20, 30, 35, 40, 45, 50, 55, 60, 65, 70, 75, 80 und 85 sowie jeweils mit einer Schwankungsbreite von bis zu plus/minus 5, bevorzugt liegt u' zwischen größer gleich 10 bis 40, bevorzugt 10 bis 35, Besonders bevorzugt liegt die Summe von (u + u') zusammen im Mittel zwischen größer gleich 5 bis 100, insbesondere zwischen größer gleich 20 bis 75, wie um 25 bis 60.

Als HX kommt Acrylsäure oder auch jede andere für die spätere Anwendung geeignete organische oder anorganische Säure in Betracht. Generell kann die in der Zusammensetzung vorliegende Acrylsäure bei Bedarf abgetrennt werden. Vorzugsweise kann sie, über Wasserstoffbrückenbindungen oder als Salz gebunden, in der Zusammensetzung verbleiben und bei einer späteren Anwendung zur Vernetzung des Produktes bspw. als Comonomer beitragen.

Beispielhaft dargestellte Acrylamido-Gruppen (Gruppe-C) an den Silicium-Atomen der Siloxanole sind nachfolgend jeweils an einem Siliciumatom exemplarisch dargestellt:

≡Si-(CH₂)_{c}-[(NH)(CH₂)_{d}]ₑ[(NH)](CH₂)_{f}]_{g}NH₍₁₋ₕ₎R³ₕ-(CO)CR⁴=CHR⁵

≡Si-(CH₂)ⱼ-NH(CH₂-CH₂-NH)-(CO)CR⁴=CHR⁵

≡Si-(CH₂)ⱼ-NH₂₋ₚ(CH₂-CH₂-NH-(CO)CR⁴=CHR⁵)ₚ

Grundsätzlich sind unter einer Acrylamido-Gruppe (Gruppe-C), insbesondere einer Acrylamido-Gruppe der Siloxanole, alle denkbaren Umsetzungen der genannten Aminoalkyl-funktionellen Gruppen mit (Meth)acrylsäureanhydrid bzw. CHR⁵=CR⁴(CO)- zu verstehen, insbesondere aber die aus einer Umsetzung einer Amino-funktionellen Gruppe-B gemäß der Formel II und/oder III mit einem Acrylsäureanhydrid der Formel IV. Somit kann eine Gruppe C ausgewählt sein aus

-(CH₂)_{c}-[(NH)(CH₂)_{d}]ₑ[(NH)](CH₂)_{f}]_{g}NH₍₁₋ₕ₎R³ₕ-(CO)CR⁴=CHR⁵,

-(CH₂)ⱼ-NH(CH₂-CH₂-NH)-(CO)CR⁴=CHR⁵

und

-(CH₂)ⱼ-NH₂₋ₚ(CH₂-CH₂-NH-(CO)CR⁴=CHR⁵)ₚ.

Gleichfalls Gegenstand der Erfindung ist ein Verfahren zur Herstellung einer Zusammensetzung, umfassend Acrylamido-funktionelle Siloxanole, insbesondere im Wesentlichen wasserlösliche Acrylamido-funktionelle Siloxanole, sowie Zusammensetzungen erhältlich nach diesem Verfahren, indem
- das Verfahren in mindestens einem Schritt in Gegenwart einer definierten Menge Wasser von 0,1 bis 2,0 mol Wasser pro Mol Siliciumatome durchgeführt wird, und
- eine Komponente A, eine Aminoalkyl-funktionelle Siliciumverbindung, ausgewählt ist aus:
   (i) mindestens einem Aminoalkyl-funktionellen Alkoxysilan oder einem Gemisch von Aminoalkyl-funktionellen Alkoxysilanen der Formel I, definiert wie vorstehend, oder
   (ii) einen Hydrolyse- oder Kondensationsprodukt mindestens eines Aminoalkyl-funktionellen Alkoxysilans der Formel I oder
   (iii) einen Gemisch umfassend mindestens ein Aminoalkyl-funktionelles Alkoxysilan der Formel I und einem Hydrolyse- und/oder Kondensations produkt mindestens eines Aminoalkyl-funktionellen Alkoxysilans der Formel I,
- mit einer Komponente B, einem Acrylsäureanhydrid der Formel IV, definiert wie vorstehend, insbesondere Methacrylsäureanhydrid oder das (unsubstituierte) Acrylsäureanhydrid, umgesetzt wird, und optional
- der bei der Umsetzung gebildete Hydrolysealkohol zumindest teilweise entfernt wird. Vorzugsweise wird die Umsetzung in Gegenwart eines Verdünnungsmittels durchgeführt, wobei ein organisches protisches Verdünnungsmittel wie Alkohol bevorzugt ist.

Dabei ist es bevorzugt, wenn die definierte Menge Wasser in einem Verfahrensschritt vor dem Schritt der Umsetzung mit der Komponente B eingestellt wird, insbesondere zur Herstellung der Komponenten A (ii) oder (iii) aus (i).

Erfindungsgemäß ist es nicht notwendig, die erhaltenen Zusammensetzungen weiter aufzureinigen, insbesondere ist eine aufwendige destillative Aufarbeitung der Acrylamido-funktionellen Siloxanole nicht notwendig, denn vorzugsweise können direkt die Sumpfprodukte verwendet werden. Die erfindungsgemäßen Sumpfprodukte bedürfen keiner weiteren Aufreinigung, weil keine störenden Katalysatoren oder störenden Stabilisatoren in den Sumpfprodukten enthalten sind. Folglich lassen sich die erfindungsgemäßen Zusammensetzungen viel wirtschaftlicher und mit umweltverträglicheren Ausgangssubstanzen herstellen als es im Stand der Technik beschrieben ist.

Ein besonderer Vorteil des erfindungsgemäßen Verfahrens ist, dass keine Gasphasenstabilisatoren verwendet werden müssen, wie es im Stand der Technik notwendig ist, denn die erfindungsgemäße Verfahrensführung erlaubt es die Zusammensetzung direkt als Sumpfprodukt zu verwenden. Eine aufwändige Rektifikation der Produkte wie im Stand der Technik kann unterbleiben. Folglich lassen sich die erfindungsgemäßen Zusammensetzungen viel wirtschaftlicher und mit umweltverträglicheren Ausgangssubstanzen herstellen als es im Stand der Technik beschrieben ist.

Ein weiterer Vorteil der erfindungsgemäßen Zusammensetzungen ist, dass sie beim Anwender kürzere Vorbereitungszeiten vor der Anwendung ermöglichen. So kann eine erfindungsgemäße Zusammensetzung beim Anwender einfach mit Wasser auf die gewünschte Konzentration gebracht werden; sie löst sich spontan auf und bildet eine klare Lösung. Einfaches Rühren beschleunigt die Auflösung in Wasser. Auf die Zugabe von weiteren Chemikalien, wie im Stand der Technik, Säure etc., kann verzichtet werden.

Gemäß bevorzugter Ausführungsformen wird das Verfahren vorzugsweise mit einer Aminoalkyl-funktionellen Siliciumverbindung, ausgewählt aus einem Aminoalkyl-funktionelle Alkoxysilan der Formel I, oder einem Hydrolyse- oder Kondensationsprodukt mindestens eines Aminoalkyl-funktionellen Alkoxysilans der Formel I oder einem Gemisch, umfassend mindestens ein Aminoalkyl-funktionelles Alkoxysilan der Formel I und einem Hydrolyse- und/oder Kondensationsprodukt mindestens eines Aminoalkyl-funktionellen Alkoxysilans der Formel I durchgeführt, wobei die Hydrolyse und/oder Kondensation des Aminoalkyl-funktionellen Alkoxysilans der Formel I in Gegenwart einer definierten Menge Wasser erfolgt, vorzugsweise entspricht die definierte Menge Wasser 0,1 bis 2,0 mol Wasser pro Mol Siliciumatome der im Verfahren eingesetzten Aminoalkyl-funktionellen Siliciumverbindung, insbesondere der Formel I, bevorzugt sind 0,3 bis 1,5 mol Wasser pro Mol Siliciumatome der vorgenannten Siliciumverbindung, besonders bevorzugt sind 0,5 bis 1,0 mol Wasser pro Mol Siliciumatome der Siliciumverbindung, vorzugsweise wird die definierte Menge Wasser in einem Verfahrensschritt vor dem Schritt der Umsetzung mit der Komponente B eingestellt und vorzugsweise zumindest teilweise durch die Hydrolyse aufgebraucht.

Gemäß bevorzugter Ausführungsformen wird das Verfahren vorzugsweise mit einem Aminoalkyl-funktionellen Alkoxysilan der Formel I, oder einem Hydrolyse- oder Kondensationsprodukt mindestens eines Aminoalkyl-funktionellen Alkoxysilans der Formel I oder einem Gemisch umfassend mindestens ein Aminoalkyl-funktionelles Alkoxysilan der Formel I und einem Hydrolyse- und/oder Kondensationsprodukt mindestens eines Aminoalkyl-funktionellen Alkoxysilans der Formel I durchgeführt,
a) mit R¹ unabhängig gleich Methyl oder Ethyl und mit a = 0 und b = 0 mit c = 1, 2 oder 3 und mit der Gruppe-B gemäß Formel II mit g = 0 und e = 1 und h = 0, d = 1, 2, 3, vorzugsweise ist d = 2, oder
b) mit R¹ unabhängig gleich Methyl oder Ethyl und mit a = 0 und b = 0 mit c = 3 und mit der Gruppe-B gemäß Formel II mit g, e und h jeweils gleich 0 oder gemäß einer Alternative mit a = 0, b = 0, c = 3, und mit der Gruppe-B gemäß Formel II mit e = 1, d = 1, 2, 3, vorzugsweise ist d = 2 und mit g = 0, h = 0 oder mit der Gruppe-B gemäß Formel II mit e = g = 0 oder 1, und d = f = 2 oder 3 und h = 0 mit c = 3 oder mit der Gruppe-B gemäß Formel III mit j = 3 und p gleich 1 oder 2, oder
c) mit R¹ unabhängig gleich Methyl oder Ethyl und mit a = 0 und b = 0 mit c = 2 und mit der Gruppe-B gemäß Formel II mit g, e und h jeweils gleich 0 oder gemäß einer Alternative mit a = 0, b = 0, c = 3, und mit der Gruppe-B gemäß Formel II mit e = 1, d = 1, 2, 3, vorzugsweise ist d = 2 und mit g = 0, h = 0 oder mit der Gruppe-B gemäß Formel II mit e = g = 0 oder 1, und d = f = 2 oder 3 und h = 0 mit c = 2 oder mit der Gruppe-B gemäß Formel III mit j = 3 und p gleich 1 oder 2, oder
d) mit R¹ unabhängig gleich Methyl oder Ethyl und mit a = 0 und b = 0 mit c = 1 und mit der Gruppe-B gemäß Formel II mit g, e und h jeweils gleich 0 oder gemäß einer Alternative mit a = 0, b = 0, c = 3, und mit der Gruppe-B gemäß Formel II mit e = 1, d = 1, 2, 3, vorzugsweise ist d = 2 und mit g = 0, h = 0 oder mit der Gruppe-B gemäß Formel II mit e = g = 0 oder 1, und d = f = 2 oder 3 und h = 0 mit c = 1 oder mit der Gruppe-B gemäß Formel III mit j = 3 und p gleich 1 oder 2.

Ebenfalls ist es bevorzugt, wenn das Verfahren vorzugsweise mit einem Aminoalkyl-funktionellen Alkoxysilan, oder einem Hydrolyse- oder Kondensationsprodukt mindestens eines Aminoalkyl-funktionellen Alkoxysilans oder einem Gemisch umfassend mindestens ein Aminoalkyl-funktionelles Alkoxysilan und ein Hydrolyse- und/oder Kondensationsprodukt mindestens eines Aminoalkyl-funktionellen Alkoxysilans ausgewählt aus den folgenden Aminoalkyl-funktionellen Alkoxysilanen, insbesondere der allgemeinen Formel I durchgeführt wird: 3-Aminopropyltrimethoxysilan, 3-Aminopropyltriethoxysilan, 3-Aminopropylmethyldimethoxysilan, 3-Aminopropylmethyldiethoxysilan, 1-Aminomethyltrimethoxysilan, 1-Aminomethyltriethoxysilan, 2-Aminoethyltrimethoxysilan, 2-Aminoethyltriethoxysilan, 3-Aminoisobutyltrimethoxysilan, 3-Aminoisobutyltriethoxysilan, N-n-Butyl-3-aminopropyltriethoxysilan, N-n-Butyl-3-aminopropylmethyldiethoxysilan, N-n-Butyl-3-aminopropyltrimethoxysilan, N-n-Butyl-3-aminopropylmethyldimethoxysilan, N-n-Butyl-1-amino-methyltriethoxysilan, N-n-Butyl-1-aminomethylmethyldimethoxysilan, N-n-Butyl-1-aminomethyltrimethoxysilan, N-n-Butyl-1-aminomethylmethyltriethoxysilan, Benzyl-3-aminopropyltrimethoxysilan, Benzyl-3-aminopropyltriethoxysilan, Benzyl-2-aminoethyl-3-aminopropyltrimethoxysilan, Benzyl-2-aminoethyl-3-aminopropyltriethoxysilan, Diaminoethylen-3-propyltrimethoxysilan, Diaminoethylen-3-propyltriethoxysilan, Triaminodiethylen-3-propyltrimethoxysilan, Triaminodiethylen-3-propyltriethoxysilan, (2-Aminoethylamino)-ethyltrimethoxysilan, (2-Aminoethylamino)-ethyltriethoxysilan, (1-Aminoethylamino)-methyltrimethoxysilan und (1-Aminoethylamino)-methyltriethoxysilan, wobei insbesondere Di- und oder Triaminoalkoxysilane bevorzugt sind. Besonders bevorzugt sind Diaminoethylen-3-propyltrimethoxysilan, Diaminoethylen-3-propyltriethoxysilan, Triaminodiethylen-3-propyltrimethoxysilan, Triaminodiethylen-3-propyltriethoxysilan,

Weiter ist es besonders bevorzugt, wenn das Verfahren in mindestens einem Schritt in Gegenwart einer definierten Menge Wasser durchgeführt wird, insbesondere wird wie folgt die
a) Komponente A, eine Aminoalkyl-funktionelle Siliciumverbindung, die ausgewählt ist aus: (i) mindestens einem Aminoalkyl-funktionellen Alkoxysilan oder einem Gemisch von Aminoalkyl-funktionellen Alkoxysilanen der Formel I, definiert wie vorstehend, mit einer definierten Menge Wasser oder Wasser im Überschuss umgesetzt oder (ii) ein Hydrolyse- oder Kondensationsprodukt mindestens eines Aminoalkyl-funktionellen Alkoxysilans der Formel I wird aus dem Aminoalkyl-funktionellen Alkoxysilan der Formel I in Gegenwart einer definierten Menge Wasser hergestellt, (iii) ein Gemisch umfassend mindestens ein Aminoalkyl-funktionelles Alkoxysilan der Formel I und ein Hydrolyse- und/oder Kondensationsprodukt mindestens eines Aminoalkyl-funktionellen Alkoxysilans der Formel I, wird aus dem Aminoalkyl-funktionellen Alkoxysilan in Gegenwart einer definierten Menge Wasser hergestellt, und
b) nachfolgend mit einer Komponente B, einem Acrylsäureanhydrid der Formel IV, definiert wie vorstehend, insbesondere Methacrylsäureanhydrid oder das (unsubstituierte) Acrylsäureanhydrid, umgesetzt und
c) optional wird der bei der Umsetzung gebildete Hydrolysealkohol zumindest teilweise entfernt.

Gemäß weiterer bevorzugter Verfahrensvarianten sind Verfahren bevorzugt, in denen
- in einem Schritt (I) umfassend die nachfolgenden Teilschritte die Komponente A eine Aminoalkyl-funktionelle Siliciumverbindung, die vorzugsweise mindestens ein Aminoalkyl-funktionellen Alkoxysilan der Formel I ist, wie vorstehend definiert, optional in Gemisch mit einem Verdünnungsmittel, insbesondere organischen protischen Verdünnungsmittel, bevorzugt einem Alkohol, besonders bevorzugt Methanol, Ethanol oder Propanol,
- mit einer definierten Menge Wasser versetzt wird, vorzugsweise wird das Wasser kontinuierlich oder diskontinuierlich zudosiert, vorzugsweise werden 0,5 bis 1,5 mol, besonders bevorzugt 0,5 bis 1,0 mol Wasser pro Mol Siliciumatome der Siliciumverbindung zudosiert; vorzugsweise innerhalb eines definierten Zeitraumes, und vorzugsweise unter Rühren, wobei vorzugsweise im Temperaturbereich 0 bis 75 °C gehalten wird, insbesondere 30 bis 75 °C, bevorzugt zwischen 40 bis 65 °C, besonders bevorzugt zwischen 50 bis 65 °C, zwischen 10 Minuten bis 10 Stunden, vorzugsweise zwischen 10 Minuten bis 5 Stunden, besonders bevorzugt zwischen 10 Minuten bis 2,5 Stunden, und
- optional wird der Hydrolysealkohol und/oder das zugesetzte Verdünnungsmittel, vorzugsweise der zugesetzte Alkohol, zumindest teilweise entfernt, und
- zum resultierenden Gemisch wird das Acrylsäureanhydrid der Formel IV zugesetzt, insbesondere bei einer Temperatur des Gemisches zwischen 0 bis 30 °C, vorzugsweise wird das Acrylsäureanhydrid der Formel IV so zudosiert, dass die Temperatur des Gemisches nicht über 75 °C ansteigt, und
- optional wird dem Gemisch ein Stabilisator zugesetzt, und im Schritt II wird alternativ Schritt (IIa) oder Schritt (IIb) durchgeführt, wobei
- in einem Schritt (IIa) gemäß einer Alternative wird nach dem Schritt (I) der Hydrolysealkohol und/oder das zugesetzte Verdünnungsmittel zumindest teilweise unter Umgebungs- oder vermindertem Druck und erhöhter Temperatur entfernt, vorzugsweise wird im Schritt (IIa) Wasser zugesetzt, insbesondere kann das Wasser vor, während und nach weiterer Zugabe von Verdünnungsmittel zugesetzt werden, oder
- in einem Schritt (IIb) gemäß einer weiteren Alternative wird nach dem Schritt (I) dem Gemisch eine Base zugesetzt, insbesondere wenn das Acrylsäureanhydrid im molaren Überschuss zu den primären Amino-Gruppen des Aminoalkyl-funktionellen Silans vorliegt, vorzugsweise eine Base, die mit der Acrylsäure ein in Wasser oder in der alkoholischwässrigen Phase schwerlösliches Salz bildet, wie bspw. ein Metallsalze, Erdalkalihyroxid, Erdalkalioxid oder Alkalihydroxid, und optional
- Abtrennen des Niederschlages, insbesondere des schwerlöslichen Salzes des Acrylats, optional
- Zugabe einer organischen Säure und zumindest teilweises Entfernen des Hydrolysealkohols und/oder des Verdünnungsmittels sowie optional Entfernen zumindest eines Teils des Wassers, wobei optional zum Entfernen des Hydrolysealkohols in diesem Schritt weiteres Wasser zugesetzt wird. Die so erhaltene Zusammensetzung kann direkt verwendet werden, vorzugsweise wird sie mit Wasser auf den gewünschten Wirkstoffgehalt an Acrylamidofunktionellem Siloxanol eingestellt. Zur Bildung schwerlöslicher Salze können Alkali- oder Erdalkalihydroxide oder - oxide wie Calciumhydroxid, Calciumoxid aber auch Natriumhydroxid/-oxid verwendet werden. Generell können die Salze von Ca, Mg, Ba, Sr, Al und/oder Zink zur Bildung schwerlöslicher Salze verwendet werden.

Als Verdünnungsmittel kommen generell alle geeigneten Verdünnungsmittel in Betracht, wie organische aprotische oder protische Verdünnungsmittel und Gemische dieser, wie beispielhaft Alkohole, Ether oder Ketone, Essigester, Methylenchlorid, wobei organisch, protische Verdünnungsmittel oder zur Verdünnung der hergestellten Zusammensetzung Wasser bevorzugt sind. Der bereits als Verdünnungsmittel vorhandene und/oder bei der Umsetzung entstandene (Hydrolyse)alkohol wird in allen erfindungsgemäßen Verfahrensvarianten im Wesentlichen, bevorzugt vollständig, entfernt. Die destillative Abtrennung des Alkohols wird vorzugsweise unter vermindertem Druck durchgeführt. Alternativ, bis ein Alkoholgehalt von kleiner 20 Gew.-% bis 0,0001 Gew.-%, vorzugweise kleiner gleich 12 Gew.-%, bevorzugt kleiner gleich 5 Gew.-%, besonders bevorzugt kleiner gleich 3,0 Gew.-%, ganz besonders bevorzugt kleiner gleich 1,0 Gew.-%, insbesondere kleiner gleich 0,5 Gew.-% nachgewiesen wird bzw. bis hin zur aktuellen analytischen Nachweisgrenze. In der Regel ist die resultierende erfindungsgemäße Zusammensetzung dann im Wesentlichen lösemittelfrei, insbesondere alkoholfrei. Die so erhaltene Zusammensetzung entspricht bevorzugt direkt der erfindungsgemäßen Zusammensetzung, und muss vorzugsweise nicht selbst weiter aufgereinigt werden.

Besonders bevorzugt ist es, wenn das flüchtige Verdünnungsmittel und der Hydrolysealkohol bis auf einen Gehalt in der Gesamtzusammensetzung von kleiner gleich 12 Gew.-% bis 0 Gew.-% entfernt werden, vorzugsweise auf kleiner gleich 10 Gew.-%, besonders bevorzugt kleiner gleich 5 Gew.-%, ganz besonders bevorzugt kleiner gleich 2 Gew.-% bis 0,0001 Gew.-%, insbesondere kleiner gleich 1 bis 0,0001 Gew.-%, wobei das Entfernen vorzugsweise durch Destillation, insbesondere unter vermindertem Druck im Bereich von 1 bis 1000 mbar, bevorzugt von 0,001 bis 350 mbar, besonders bevorzugt zwischen 0,001 bis 250 mbar, bei einer milden Temperatur von unter 60 °C Sumpftemperatur erfolgt, insbesondere unter 55 °C.

Vorzugsweise wird in dem Verfahren das molare Verhältnis der Stickstoffatome der Aminoalkyl-funktionellen Siliciumverbindung, insbesondere der Aminoalkyl-funktionellen Silane der Formel I, zum molaren Verhältnis der aus dem Acrylsäureanhydrid der Formel IV freigesetzten CHR⁵=CR⁴(CO)- Acrylcarbonyl-Funktion im Bereich von 1 zu 5 bis 5 zu 1 liegt, insbesondere 1 zu 2 bis 2 zu 1, bevorzugt 1 zu 1,5 bis 1,5 zu 1, besonders bevorzugt um 1 zu 1 mit einer Schwankungsbreite von plus/minus 0,5, vorzugsweise plus/minus 0,2.

Alternativ kann es besonders bevorzugt sein, ein Diaminoalkyl-funktionelles Silan äquimolar mit Acrylsäureanhydrid der Formel IV einzusetzen. Dabei fungiert die sekundäre Aminofunktion zur Neutralisierung der freien Acrylsäure und kann zu einem Aminohydro(meth)acrylat abreagieren, das insbesondere nachfolgend basisch spaltbar ist.

Weiter ist ein Verfahren bevorzugt, indem der Wirkstoffgehalt an Acrylamido-funktionellen Siloxanolen auf 0,0001 bis 99,9 Gew.-% in der Gesamtzusammensetzung eingestellt wird, insbesondere auf 10 bis 80 Gew.-%, bevorzugt auf 20 bis 60 Gew.-%, besonders bevorzugt auf 35 bis 60 Gew.-%, wobei der Wirkstoffgehalt durch Verdünnung mit einem Verdünnungsmittel, vorzugsweise mit Wasser oder ggf. mit wässrigen Alkoholen oder jedem anderen geeigneten Verdünnungsmittel auf jeden Wert zwischen 99,9 Gew.-% und 0,00001 Gew.-% eingestellt werden kann.

Gleichfalls ist der Zusatz von üblichen Säuren, Basen, Additiven, Hilfsmitteln, Füllstoffen Stabilisatoren, Pigmenten zur Einstellung der Produkteigenschaften, der Farbe oder zur Erhöhung der Lagerstabilität möglich.

Nach dem erfindungsgemäßen Verfahren werden Zusammensetzungen erhalten, deren pH-Werte in der Regel nach dem Entfernen des Verdünnungsmittels, Hydrolysealkohols und zumindest Teilen des Wassers einen Wert zwischen 3 bis 11, vorzugsweise zwischen 5 bis 11 aufweisen, insbesondere zwischen 6 bis 10, bevorzugt zwischen 6 bis 8, besonders bevorzugt zwischen 7 bis 8, oder zwischen 6,5 bis 8,0 oder zwischen 8 bis 10. Dabei ist es besonders bevorzugt, wenn die hergestellten Zusammensetzungen (Meth)acrylamidoalkyl-funktionelle Siloxanole umfassen, die ohne eine Modifizierung des pH-Wertes gut wasserlöslich sind. Diese Zusammensetzungen weisen dann üblicherweise einen pH-Wert von 6 bis 9 auf.

Zusätzlich oder alternativ kann der pH-Wert der Zusammensetzung durch Zugabe einer Säure oder Base eingestellt werden. Vorzugsweise kann der pH-Wert einer Zusammensetzung auf einen pH-Wert unterhalb von 8 in wässriger Phase eingestellt werden, besonders bevorzugt zwischen 3 bis 8, insbesondere zwischen 3 bis 6, bevorzugt zwischen 3 bis 5,5, besonders bevorzugt zwischen 3 bis 5,0. Typische Säuren zur Einstellung des pH-Wertes können mineralische Säure, wie HCl, Schwefelsäure oder auch organische Säuren sein, wobei organische Säuren, wie Essigsäure, Milchsäure oder Ameisensäure bevorzugt sind.

Das Herstellverfahren wirkt sich ebenfalls vorteilhaft auf die Viskosität der Zusammensetzungen aus. So sind die erfindungsgemäßen hergestellten Zusammensetzungen leicht bewegliche Flüssigkeiten von einer Viskosität, die eine leichte Verarbeitung, einfaches Umfüllen und Abmessen erlauben. Die Viskosität der Zusammensetzungen - hergestellt als Sumpfprodukt - liegt zwischen 1 mPas bis 2000 mPas, vorzugsweise zwischen 1 bis 1500 mPas, weiter bevorzugt zwischen 1 bis 400 mPas.

Gleichfalls Gegenstand der Erfindung sind Zusammensetzungen erhältlich nach einem vorgenannten Verfahren und umfassend Acrylamido-funktionelle Siloxanole, vorzugsweise im Wesentlichen wasserlösliche Acrylamido-funktionelle Siloxanole,insbesondere Acrylamido-funktionelle Siloxanole, die zumindest teilweise bis vorzugsweise im Wesentlichen vollständig hydrolysiert sind, weiter bevorzugt umfasst die Zusammensetzung Acrylamidoalkyl-aminoalkyl-funktionelle Siloxanole und ggf. Acrylamidoalkyl-aminoalkyl-funktionelle Silanole.

Weiter ist Gegenstand der Erfindung die Verwendung einer Zusammensetzung sowie der Verfahrensprodukte als Haftvermittler, zur Funktionalisierung von Glas, insbesondere zur Funktionalisierung von Glasfasern, zur Modifizierung von Füllstoffen, Pigmenten und/oder anorganischen Oberflächen, insbesondere als Füllstoffbeschichtung, Beschichtung von Pigmenten, Beschichtung von anorganischen Oberflächen, in Zahnabformmassen, in Dentalkunststoffmassen, als Additiv in Polymeren, in Klebstoffen, in Dichtstoffen, in Faserverbundwerkstoffen, zusammen mit Polymeren, insbesondere Thermoplasten, Duroplasten, Elastomeren, zur Funktionalisierung von Polymeren, zur Einstellung der Hydrophilie von Polymeren. Besonders bevorzugt ist die Verwendung zu Herstellung wässriger Systeme umfassend die erfindungsgemäßen Acrylamido-funktionelle Siloxanole oder damit modifizierter Materialien, Artikel und/oder Produkte.

Das folgende Beispiel erläutert das erfindungsgemäße Verfahren näher, ohne die Erfindung auf dieses Beispiel zu beschränken.

### Bestimmungsmethoden:

Der Alkoholgehalt nach Hydrolyse wird gaschromatographisch bestimmt (Gew.-%). SiO₂-Gehalt von organischen Siliciumverbindungen: Wird nach dem Fachmann bekannten Verfahren bestimmt, wie bspw. Oxidieren der organischen Bestandteile. Nachfolgend Veraschung, mit Flußsäure abrauchen und Bestimmung der Gewichtsdifferenz (% = Gew.-%).

Bestimmung von Stickstoff: Nach einer dem Fachmann bekannten Methode, beispielsweise nach Kjedahl. Trübung: DIN EN ISO 7027, mit Gerät der Fa. HACH Lange, Model 2100 ISO.

### Eingesetzte Verbindungen:

"TEMPO (= 2,2,6,6-tetramethylpiperidinyloxy free radical)" und "4-hydroxyTEMPO";
"SANTONOX (Flexsys America, Akron, OH) antioxidant 4,4'-thio-bis (6-t-butyl-m-cresol)"

### Beispiel 1:

In einer 500 ml Rührapparatur mit Destillationsbrücke wurden 156,00 g N-(3-(trimethoxysilyl)propyl)ethylendiamin (0,70 mol) und 40,20 g Methanol vorgelegt. Unter Rühren wurden innerhalb von 6 Minuten 9,52 g VE-Wasser (0,53 mol) zugetropft. Die Sumpftemperatur stieg dabei auf 47,8 °C. Bei einer Sumpftemperatur von 54 °C bis 59 °C wurde 2 Stunden nachgerührt. Bei einer Sumpftemperatur von 23,1 °C wurde dann 107,9 g Methacrylsäureanhydrid (0,70 mol) innerhalb von 2 Stunden zugetropft. Dabei stieg die Sumpftemperatur auf max. 51,7 °C. In den Sumpf wurden als zusätzlicher Stabilisator 0,02 g 4-Hydroxy-Tempo gegeben. Anschließend wurden bei einem Absolutdruck von ca. 200 mbar und einer Sumpftemperatur von ca. 40 °C 30,21 g Destillat abgenommen. Der Methanol Gehalt des Destillats betrug 98,3 Flächen% (GC-WLD Bestimmung). Die Viskosität im Sumpf nahm deutlich zu. Für weiterführende Analytik wurden aus dem Sumpf 78,1 g Probe entnommen. Anschließend wurden bei einer Sumpftemperatur von 32,2 °C 99,61 g Wasser innerhalb von zwei Minuten zugegeben. Die Sumpftemperatur stieg dabei auf 36,3 °C an. Bei einem Absolutdruck von 200 mbar und einer Sumpftemperatur von ca. 49 °C wurden 45,2 g Methanol/Wasser-Gemisch abdestilliert. Nachfolgend wurden 120,02 g Wasser eingerührt und bei einem Absolutdruck von 200 mbar bis128 mbar 106,02 g Methanol/Wasser-Gemisch abdestilliert. Als Sumpfprodukt wurde eine klare leicht viskose gelbliche Flüssigkeit erhalten. Ausbeute: 227,9 g. Wie in Tabelle 1 ersichtlich hat das Produkt einen Feststoffgehalt von 54,6 %.

**Tabelle 1: Analysenergebnisse vom Sumpfprodukt aus Beispiel 1**

| Bestimmung | Methode | Ergebnis |
|---|---|---|
| Gesamt-N [%] | s.o. | 5,2 |
| Feststoffgehalt [%] | 3 g / 3 Stunden / 105 °C | 54,6 |
| SiO₂ Gehalt [%] | s.o. | 11,6 |
| pH | DIN ISO 4925 | 7,7 |
| Dichte [g/cm³] | DIN 51757 | 1,142 |
| Viskosität [mPas] | DIN 53015 | 351 |
| Methanol frei [%] | s.o. | 0,1 |
| ¹H- und ¹³C-NMR | Gefunden wurde das einfach umgesetzte oligomerisierte Zielprodukt. Amidierung ist am primären Amin erfolgt. | |

### Beispiel 2:

In einer 8 I Rührapparatur mit Destillationsbrücke wurde 2490,58 g N-(3-(trimethoxysilyl)propyl)ethylendiamin 11,2 mol) und 640,92 g Methanol vorgelegt. Unter Rühren wurden innerhalb von 11 Minuten 161,54 g VE-Wasser (8,96 mol) zugetropft. Die Sumpftemperatur stieg dabei auf 54,7 °C. Bei einer Sumpftemperatur von 51 °C bis 56 °C wurde 0,8 Stunden nachgerührt. Bei einer Sumpftemperatur von 36,3 °C wurde dann eine Lösung aus 1727,24 g Methacrylsäureanhydrid (11,2 mol) und 3,40 g 4-Hydroxy-Tempo innerhalb von 2,4 Stunden zugetropft. Dabei stieg die Sumpftemperatur auf max. 53,6 °C an. Der Sumpf bleibt farblos klar. Anschließend wurden innerhalb von 14 Minuten 2400,32 g VE-Wasser zudosiert. Bei einem Absolutdruck von ca. 200 mbar bis 112 mbar wurde freies Methanol abdestilliert. Die Sumpftemperatur betrug während der Destillation 46,0 °C bis 52,2 °C. Die Gesamtmenge Destillat betrug 2819,3 g. Während der Destillation wurde in vier Portionen insgesamt 3699,72 g VE-Wasser zugegeben. Als Sumpfprodukt wurde eine klare nur leicht gelblich gefärbte niedrigviskose Flüssigkeit erhalten. Ausbeute: 8082,3 g. Wie in Tabelle 2 ersichtlich hat das Produkt einen Feststoffgehalt von 40,6 %.

**Tabelle 2: Analysenergebnisse vom Sumpfprodukt aus Beispiel 2**

| Bestimmung | Methode | Ergebnis |
|---|---|---|
| Gesamt-N [%] | s.o. | 4,1 |
| Feststoffgehalt [%] | 3 g / 3 Stunden / 105 °C | 40,6 |
| SiO₂ Gehalt [%] | s.o. | 8,3 |
| pH | DIN ISO 4925 | 7,1 |
| Dichte [g/cm³] | DIN 51757 | 1,097 |
| Viskosität [mPas] | DIN 53015 | 20 |
| Methanol frei [%] | s.o. | 1,4 |
| ¹H- und ¹³C-NMR | Gefunden wurde das einfach umgesetzte oligomerisierte Zielprodukt. Amidierung ist am primären Amin erfolgt. | |

### Vergleichsbeispiel 1 (Vergleichsbeispiel zu WO 00/75148 A1):

In einer 1 I Rührapparatur mit Destillationsbrücke wurden 398,07 g Aminopropyltriethoxysilan (1,8 mol) vorgelegt, 1,99 g Dibutylzinnoxid, 0,037 g lonol und 0,18 g 4,4'-Thiobis(6-Tertiärbutyl-m-cresol) zugerührt. Anschließend wurde innerhalb von 2 Stunden eine Mischung aus 360,35 g Methylmethacrylat (3,60 mol) und 5,41 g Dipropylamin bei einer Sumpftemperatur von 152,8 °C bis 165,5 °C zudosiert. Nach 0,3 Stunden Reaktionszeit wurde bei einer Kopftemperatur von 76,5 °C bis 80,4 °C ein Gemisch aus Methanol, Ethanol, Methylmethacrylat und Ethylmethacrylat abgenommen. Nach 2,5 Stunden Destillationszeit wurden bei einem Absolutdruck von 316 mbar bis < 1 mbar und einer Sumpftemperatur bis 157,2 °C Restmengen an Leichtsieder aus dem Sumpfprodukt entfernt. Insgesamt wurden 287,8 g Destillat abgenommen. Als Sumpfprodukt wurden 461,35 g leicht gelbliche niedrigviskose Flüssigkeit erhalten. Ausweislich der Offenbarung der WO 00/75148 A1 wird das Methacryl-Rohprodukt im Hochvakuum destilliert. Für die Zwecke der Bestimmung der Löslichkeit war es vorliegend ausreichend das Rohprodukt zu verwenden, das noch Dibutylzinnoxid enthält. Für eine spätere Anwendung wäre eine gemäß der WO 00/75148 A1 offenbarte Rektifikation notwendig.

**Tabelle 3: Analysenergebnisse aus Vergleichsbeispiel 1**

| Bestimmung | Methode | Ergebnis |
|---|---|---|
| Gesamt-N [%] | s.o. | 5,0 |
| SiO₂ Gehalt [%] | s.o. | 22,0 |
| Freies Methanol [%] | s.o. | 0,1 |
| pH | DIN ISO 4925 | 9,7 |
| Viskosität [mPas] | DIN 53015 | 50,1 |

### Löslichkeitsverhalten:

Tabelle 4 zeigt das Löslichkeitsverhalten in Abhängigkeit des eingesetzten Aminosilans bei der Umsetzung mit Methacrylsäureanhydrid. Sumpfprodukte aus Beispiel 1 und Beispiel 2 zeigen auch bei direktem Lösen in VE-Wasser (ohne Zugabe von Essigsäure) eine gute Löslichkeit (löst sich innerhalb von wenigen Sekunden spontan auf und führt zu klaren Mischungen). Das Sumpfprodukt aus Beispiel 2 zeigt auch bei deutlich erhöhten Konzentrationen ein gutes Löslichkeitsverhalten. Im Vergleich dazu löst sich das Produkt aus Vergleichsbeispiel 1 wie auch das kommerziell erhältliche Y-5997 (Momentive Performance Materials, Mischung von 2-Methacrylamidoalkoxypropylsilan mit Ethoxy und Methoxy-Gruppen als Alkoxy-Gruppen) nicht in VE-Wasser (Tabelle 6). Nur bei niedrigen pH Werten zeigt dieses Produkt nach 10 Minuten intensiven Rührens eine Löslichkeit (Tabelle 7).

**Tabelle 4: Übersicht der Löslichkeitsversuche der Sumpfprodukte (3,0% Sumpfprodukt in VE-Wasser).**

| Sumpfprodukt aus Beispiel | pH (Hydrolysat) | Trübung [FNU] | | |
|---|---|---|---|---|
| | | nach 1min. | nach 1h | nach 24h |
| 1 (Edukt: N-(3-(trimethoxysilyl)propyl)ethylendiamin | 7,3 | Klar | Klar (0,3 FNU) | Klar (0,5 FNU) - nach 6d RT Lagerung |
| 1 | 4,1¹⁾ | Klar | Klar (0,3 FNU) | Klar (1,0 FNU) - nach 7d RT Lagerung |
| 2 (Edukt: N-(3-(trimethoxysilyl)propyl)ethylendiamin | 5,4¹⁾ | Klar | Klar (1,1 FNU) | Klar (0,9 FNU) |

| | | | | |
|---|---|---|---|---|
| 1) Der pH Wert des Hydrolysats wurde durch Zugabe von Essigsäure eingestellt. | | | | |

**Tabelle 5: Übersicht der Löslichkeitsversuche vom Sumpfprodukt aus Beispiel 2**

| Hydrolysat | | | Trübung [FNU] | |
|---|---|---|---|---|
| w(Sumpfprodukt) [%] | w(H₂O) [%] | pH | 1min. | 24h |
| 6 | 94 | 6,8 | 0,7 (klar) | 0,6 (klar) |
| 12 | 88 | 6,7 | 0,9 (klar) | 0,9 (klar) |

| | | | | |
|---|---|---|---|---|
| * w = weight | | | | |

**Tabelle 6: Löslichkeit von 3% des Produktes aus Vergleichsbeispiel 1 sowie Y-5997 in VE-Wasser**

| Produkt | pH (Hydrolysat) | Trübung [FNU] | | |
|---|---|---|---|---|
| | | nach 10min. | nach 1h | nach 24h |
| Y-5997 | 9,1 | nicht gelöst | Trüb/Ausfällungen | Trüb/Ausfällungen |
| Aus Beispiel 3 | Nicht bestimmt | nicht gelöst | Trüb/Ausfällungen | Trüb/Ausfällungen |

**Tabelle 7: Löslichkeit von 3 % Y-5997 in VE-Wasser. Der pH Wert des Hydrolysates wurde durch Zugabe von Essigsäure eingestellt.**

| pH (Hydrolysat) | Trübung [FNU] | | | |
|---|---|---|---|---|
| | nach 1min. | nach 10min. | nach 1h | nach 24h |
| 4,1 | Trüb | Klar | Klar (0,3 TE/F) | Klar (1,7 FNU) - nach 7d RT Lagerung |

### VOC Freisetzung in Abhängigkeit der Wirkstoffkonzentration:

Wie in Tabelle 8 ersichtlich zeigt das Y-5997 bei einer 40%igen Wirkstoffkonzentration eine maximale VOC Freisetzung von 18 %. Das Methacrylamidopropylsiloxanol aus Beispiel 2 setzt bei gleicher Wirkstoffkonzentration nur max. 1,4 % VOC frei.

**Tabelle 8, Vergleich maximale VOC Freisetzung in Abhängigkeit der Wirkstoffkonzentration**

| Wirkstoffkonzentration [w/w%] | VOC [w/w%] | |
|---|---|---|
| | Y-5997 | Methacrylamidopropylsiloxan aus Beispiel 2 |
| 40 | 18 | 1,4 |
| 20 | 9,0 | 0,7 |
| 3 | 1,35 | 0,11 |

| | | |
|---|---|---|
| * w = weight | | |

**Tabelle 9: Berechnung der maximalen VOC Freisetzung**

| Bestimmung | Methode | Einheit | Methacrylamido-propylsiloxan aus Beispiel 2 | Y-5997 |
|---|---|---|---|---|
| Methanol nach Hydrolyse | s.o. | w/w% | 1,4 | 34 |
| Ethanol nach Hydrolyse | s.o. | w/w% | <0,1 | 11 |
| VOC | Summe aus Methanol / Ethanol nach Hydrolyse | w/w% | 1,4 | 45 |

| | | | | |
|---|---|---|---|---|
| * w = weight | | | | |

**Löslichkeit und maximale VOC Freisetzung in Abhängigkeit der Wirkstoffkonzentration:**

Wie in Tabelle 10 ersichtlich zeigt das Y-5997 bei höheren Wirkstoffkonzentrationen in Wasser/Essigsäure eine schlechte Löslichkeit. Der Zusatz von Essigsäure hilft das Y-5997 bei geringen Wirkstoffkonzentrationen in Wasser zu lösen. Dazu muss das Hydrolysat jedoch 8 Minuten intensiv gerührt werden. Das Methacrylamidopropylsiloxanol aus Beispiel 2 zeigt auch bei hoher Wirkstoffkonzentration eine spontane Löslichkeit. Ein Zusatz von Essigsäure ist nicht notwendig (siehe Tabelle 11).

**Tabelle 10: Löslichkeit und maximale VOC Freisetzung von Y-5997 in Abhängigkeit von der Wirkstoffkonzentration**

| Wirkstoffkonzentration [w/w%] | VOC [w/w%] | Löseverhalten |
|---|---|---|
| 40 | 18 | Produkt ist in VE-Wasser nicht löslich. In 1,50%iger Essigsäure bleibt das Hydrolysat jedoch noch deutlich trüb |
| 20 | 9,0 | Produkt ist in VE-Wasser nicht löslich. In 1,50%iger Essigsäure bleibt das Hydrolysat jedoch noch deutlich trüb |
| 3 | 1,35 | In 1,50%iger Essigsäure wird nach 8min. intensivem Rühren eine klares Hydrolysat erhalten |

| | | |
|---|---|---|
| * w = weight | | |

**Tabelle 11: Löslichkeit und maximale VOC Freisetzung von Methacrylamidopropylsiloxanol (Beispiel 2) in Abhängigkeit von der Wirkstoffkonzentration**

| Wirkstoffkonzentration [w/w%] | VOC [w/w%] | Löseverhalten |
|---|---|---|
| 40 | 1,40 | Produkt wurde nicht verdünnt |
| 20 | 0,70 | Löst sich spontan in Wasser: klare Flüssigkeit, unverändert nach 4d |
| 8 | 0,28 | Löst sich spontan in Wasser: klare Flüssigkeit, unverändert nach 4d |
| 3 | 0,11 | Löst sich spontan in Wasser: klare Flüssigkeit, unverändert nach 4d |

| | | |
|---|---|---|
| * w = "weight" | | |

### Beispiel 3:

In einer 1l Rührapparatur mit Destillationsbrücke wurden 251,08 g Dynasylan® TRIAMO (4,7,10-Triazedecyl-trimethoxysilan, 1,0mol) und 80,00 g Methanol vorgelegt. Unter Rühren wurden innerhalb von 2 Minuten 14,42 g VE-Wasser (0,8 mol) zugetropft. Die Sumpftemperatur stieg dabei von 36,8°C auf 41,5 °C. Bei einer Sumpftemperatur von 63-65 °C wurde 1 Stunde nachgerührt. Anschließend wurde der Sumpf auf 26,5 °C abgekühlt und innerhalb von 1,5 Stunden 215,84 g Methacrylsäureanhydrid (1,0 mol) zudosiert. Dabei stieg die Sumpftemperatur auf max. 56,0 °C. In den Sumpf wurden als zusätzlicher Stabilisator 0,42 g 4-Hydroxy-Tempo gegeben (vor der Methacrylsäureanhydrid Zugabe). Es wurde eine Sumpfprobe (35,0 g) für analytische Untersuchungen abgenommen. 300,34 g VE-Wasser wurden in den Sumpf innerhalb von 3 Minuten dosiert. Bei einem Absolutdruck von ca. 180 mbar und einer Sumpftemperatur von ca. 42 °C wurden 430,5 g Destillat (Methanol / Wasser Gemisch) abgenommen. Während der Destillation wurden insgesamt 451,18 g VE-Wasser in Sumpf eingerührt. Zum Schluss der Destillation betrug die Sumpftemperatur 52 °C bei einem Absolutdruck von 100mbar. Als Sumpfprodukt wurde eine klare leicht gelbliche Flüssigkeit erhalten.

### Ausbeute: 818,7 g

Wie aus Tabelle 12 ersichtlich, hat das Produkt einen Feststoffgehalt von 39,1 %. Es löst sich spontan in Wasser (siehe Tabelle 13)

**Tabelle 12: Analysenergebnisse bezüglich Beispiel 3**

| Bestimmung | Methode | Ergebnis |
|---|---|---|
| Gesamt-N [%] | s.o. | 4,2 |
| Feststoffgehalt [%] | 3 g / 8 Stunden / 125 °C | 39,1 |
| SiO₂ Gehalt [%] | AN-SAA 1171 | 6,7 |
| pH | DIN ISO 4925 | 6,1 |
| Dichte [g/cm³] | DIN 51757 | 1,106 |
| Viskokität [mPas] | DIN 53015 | 180 |
| Methanol frei [%] | In Anlehnung an SAA0272 | 0,2 |
| ¹H- und ¹³C-NMR | Umsetzungsgrad der Amidierung: ca. 80mol%, Zielprodukt liegt als Oligomer vor | |

**Tabelle 13: Übersicht der Löslichkeitsversuche bezüglich Beispiel 3**

| Hydrolysat | | | Trübung [FNU] | |
|---|---|---|---|---|
| w(Sumpfprodukt) [%] | w(H₂O) [%] | pH | 1min. | 24h |
| 6 | 94 | 6,8 | 2,9 (klar) | 1,6(klar) |
| 12 | 88 | 6,7 | 3,1 (klar) | 2,7(klar) |

| Hydrolysat | | | Trübung [FNU] | |
|---|---|---|---|---|
| w(Sumpfprodukt) [%] | w(0,5%ige Essigsäure) [%] | p H | 1min. | 24h |
| 6 | 94 | 5,1 | 1,7 (klar) | 1,7(klar) |
| 12 | 88 | 4,2 | 1,7 (klar) | 2,6(klar) |

### Beispiel 4:

In einer 1l Rührapparatur mit Destillationsbrücke wurden 332,07 g 3-Aminopropyl-triethoxysilan (1,50mol) und 81,04 g Ethanol vorgelegt. Unter Rühren wurden innerhalb von 3 Minuten 21,6 g VE-Wasser (1,2 mol) zugetropft. Die Sumpftemperatur stieg dabei von 32,3 °C auf 33,5 °C. Bei einer Sumpftemperatur von ca. 60 °C wurde 1 Stunde nachgerührt. Anschließend wurde der Sumpf auf 28,9 °C abgekühlt und innerhalb von 36 Minuten 77,1 g Methacrylsäureanhydrid (0,5 mol) zudosiert. Dabei stieg die Sumpftemperatur auf max. 54 °C. In den Sumpf wurden als zusätzlicher Stabilisator 0,42 g 4-Hydroxy-Tempo gegeben (vor der Methacrylsäureanhydrid Zugabe). 300,11 g VE-Wasser wurden in den Sumpf innerhalb von 3 Minuten dosiert. Für analytische Untersuchungen wurde eine Sumpfprobe (56,3 g) entnommen. Durch Zugabe von 27,30 g Eisessig wurde ein pH von ca. 7,9 erreicht. Bei einem Absolutdruck von ca. 146 mbar und einer Sumpftemperatur von ca. 40 °C wurden 390,9 g Destillat (Ethanol / Wasser Gemisch) abgenommen. Während der Destillation wurden insgesamt 50 g VE-Wasser und 121,6 g Eisessig eingerührt. Zum Schluss der Destillation betrug die Sumpftemperatur 50 °C bei einem Absolutdruck von 170 mbar. Als Sumpfprodukt wurde eine leicht trübe gelbliche Flüssigkeit erhalten, die über einen Druckfilter filtriert wurde.

Ausbeute: 531,6 g gelbliche leicht trübe Flüssigkeit.
Das Produkt löst sich spontan in Wasser.

**Tabelle 14: Analsenerebnis bezüglich Beispiel 4**

| Bestimmung | Methode | Ergebnis |
|---|---|---|
| Gesamt-N [%] | s.o. | 3,4 |
| Feststoffgehalt [%] | 3 g /19 Stunden / 125 °C | 42,4 |
| SiO₂ Gehalt [%] | AN-SAA 1171 | 14,7 |
| pH | DIN ISO 4925 | 4,4 |
| Dichte [g/cm³] | DIN 51757 | 1,145 |
| Viskokität [mPas] | DIN 53015 | 76 |
| Ethanol nach Hydrolyse [%] | In Anlehnung an SAA0272 | 1,6 |
| ¹H- und ¹³C-NMR | Umsetzungsgrad der Amidierung: >90 mol%, Zielprodukt liegt als Oligomer vor | |

## Patentansprüche

1. Zusammensetzung umfassend Acrylamido-funktionelle Siloxanole, die abgeleitet sind aus einer
a) Umsetzung einer Komponente A, die eine Aminoalkyl-funktionalisierte Siliciumverbindung ist, ausgewählt aus
(i) einem Aminoalkyl-funktionellen Alkoxysilan der Formel I oder einem Gemisch von Aminoalkyl-funktionellen Alkoxysilanen der Formel I, jeweils in Gegenwart einer definierten Menge Wasser umgesetzt wird,
oder
(ii) einem Hydrolyse- oder Kondensationsprodukt mindestens eines Aminoalkyl-funktionellen Alkoxysilans der Formel I
oder
(iii) einem Gemisch umfassend mindestens ein Aminoalkyl-funktionelles Alkoxysilan der Formel I und einem Hydrolyse- und/oder Kondensationsprodukt mindestens eines Aminoalkyl-funktionellen Alkoxysilans der Formel I,
wobei das Aminoalkyl-funktionelle Alkoxysilan der Formel I entspricht
(R¹O)_{3-a-b}(R²)ₐSi(B)_{1+b} (I)
und die Gruppe-B in Formel I unabhängig einer Gruppe der Formel II
-(CH₂)_{c}-[(NH)(CH₂)_{d}]ₑ[(NH)(CH₂)_{f}]_{g}NH₍₂₋ₕ₎ R³ₕ (II)
entspricht, in Formel I mit R¹ unabhängig eine lineare, verzweigte oder cyclische Alkyl-Gruppe mit 1 bis 8 C-Atomen, R² unabhängig eine lineare, verzweigte oder cyclische Alkyl-Gruppe mit 1 bis 8 C-Atomen, und in Formel II mit R³ unabhängig eine lineare, verzweigte oder cyclische Alkyl-, Aryl- oder Alkylaryl-Gruppe mit 1 bis 8 C-Atomen; und in Formel I ist a unabhängig gleich 0 oder 1, b unabhängig gleich 0 oder 1, in Formel II ist c unabhängig ausgewählt aus 1, 2, 3, 4, 5 und 6, d ist unabhängig ausgewählt aus 1, 2, 3, 4, 5 und 6, e ist unabhängig ausgewählt aus 0, 1, 2, 3, 4, 5 und 6, f ist unabhängig ausgewählt aus 1, 2, 3, 4, 5 und 6, g ist unabhängig ausgewählt aus 0, 1, 2, 3, 4, 5 und 6, und h ist unabhängig 0 oder 1, oder die Gruppe-B in Formel I entspricht einer Gruppe der Formel III
-(CH₂)ⱼ-NH₂₋ₚ(CH₂-CH₂-NH₂)ₚ (III)
mit j = 1, 2 oder 3 und p = 0, 1 oder 2, bevorzugt ist p ausgewählt aus 0 und 1,
und nachfolgend mit einer Komponente B, die ein Acrylsäureanhydrid ist, umgesetzt wird und optional
b) Entfernen zumindest eines Teils des Hydrolysealkohols, wobei optional zum Entfernen des Hydrolysealkohols in diesem Schritt weiteres Wasser zugesetzt wird.

2. Zusammensetzung nach Anspruch 1, **dadurch gekennzeichnet, dass** das Acrylsäureanhydrid der Formel IV entspricht,
(CHR⁵=CR⁴CO)₂O (IV),
wobei R⁴ unabhängig ein Wasserstoffatom oder eine Methyl-Gruppe und R⁵ unabhängig ein Wasserstoffatom oder eine Methyl-Gruppe ist, vorzugsweise ist R⁵ ein Wasserstoffatom.

3. Zusammensetzung nach Anspruch 1 oder 2, **dadurch gekennzeichnet, dass** mindestens ein wasserlösliches Acrylamido-funktionelles Siloxanol idealisiert der allgemeinen Formel V entspricht,
(R¹O)[(R¹O)₁₋ₐ(R²)ₐSi(C)_{1+b}O]ᵤ[(Y)Si(C)_{1+b}O]_{u'}R¹•(HX)_{z} (V),
wobei
- C einer Acrylamido-Gruppe entspricht und
- Y entspricht OR¹ oder in vernetzten und/oder raumvernetzten Strukturen unabhängig voneinander OR¹ oder O_{1/2},
- wobei R¹ Wasserstoff entspricht und R² einer linearen, verzweigten oder cyclischen Alkyl-Gruppe mit 1 bis 8 C-Atomen,
- HX eine Säure darstellt, wobei X ein anorganischer oder organischer Säure-Rest ist,
- mit jeweils unabhängig a gleich 0 oder 1, jeweils unabhängig b gleich 0 oder 1, vorzugsweise ist b gleich 0, mit jeweils unabhängig eine ganze Zahl u größer gleich 2, u' größer gleich 0 und z größer gleich 0 und (u+u') größer gleich 2,
- wobei die Zusammensetzung frei von Verdünnungsmitteln ist und beim Vernetzen keinen Alkohol mehr freisetzt, insbesondere umfasst die Zusammensetzung wasserlösliche Acrylamido-funktionelle Siloxanole, insbesondere entspricht C einer Acrylamido-Gruppe ausgewählt aus -(CH₂)_{c}-[(NH)(CH₂)_{d}]ₑ[(NH)(CH₂)_{f}]_{g}NH₍₁₋ₕ₎R³ₕ-(CO)CR⁴=CHR⁵, -(CH₂)ⱼ-NH(CH₂-CH₂-NH)-(CO)CR⁴=CHR⁵ und -(CH₂)ⱼ-NH₂₋ₚ(CH₂-CH₂-NH-(CO)CR⁴=CHR⁵)ₚ, wobei c, d, e, f, g, h, j, p sowie R³, R⁴, R⁵ wie vorstehend definiert sind.

4. Verfahren zur Herstellung einer Zusammensetzung umfassend Acrylamido-funktionelle Siloxanole, indem
- das Verfahren in mindestens einem Schritt in Gegenwart einer definierten Menge Wasser von 0,1 bis 2,0 mol Wasser je Mol Siliciumatome durchgeführt wird, und
- eine Komponente A, eine Aminoalkyl-funktionelle Siliciumverbindung, ausgewählt aus
(i) mindestens einem Aminoalkyl-funktionellen Alkoxysilan oder einem Gemisch von Aminoalkyl-funktionellen Alkoxysilanen der Formel I, definiert wie in Anspruch 1, oder
(ii) einem Hydrolyse- oder Kondensationsprodukt mindestens eines Aminoalkyl-funktionellen Alkoxysilans der Formel I
oder
(iii) einem Gemisch umfassend mindestens ein Aminoalkyl-funktionelles Alkoxysilan der Formel I und einem Hydrolyse- und/oder Kondensations produkt mindestens eines Aminoalkyl-funktionellen Alkoxysilans der Formel I,
- mit einer Komponente B, einem Acrylsäureanhydrid der Formel IV, definiert wie in Anspruch 2, umgesetzt wird und optional
- der bei der Umsetzung gebildete Hydrolysealkohol zumindest teilweise entfernt wird.

5. Verfahren nach Anspruch 4 **gekennzeichnet durch** ein Aminoalkyl-funktionelles Alkoxysilan der Formel I mit R¹ unabhängig gleich Methyl oder Ethyl und mit a = 0 und b = 0 mit c = 1, 2 oder 3 und mit der Gruppe-B gemäß Formel II mit g = 0 und e = 1 und h = 0, d = 1, 2, 3, vorzugsweise ist d = 2.

6. Verfahren nach Anspruch 4 **gekennzeichnet durch** ein Aminoalkyl-funktionelles Alkoxysilan der Formel I mit R¹ unabhängig gleich Methyl oder Ethyl und mit a = 0 und b = 0 mit c = 3 und mit der Gruppe-B gemäß Formel II mit g, e und h jeweils gleich 0 oder gemäß einer Alternative mit a = 0, b = 0, c = 3, und mit der Gruppe-B gemäß Formel II mit e = 1, d = 1, 2, 3, vorzugsweise ist d = 2 und mit g = 0, h = 0 oder mit der Gruppe-B gemäß Formel II mit e = g = 0 oder 1, und d = f = 2 oder 3 und h = 0 mit c = 3 oder mit der Gruppe-B gemäß Formel III mit j = 3 und p gleich 1 oder 2.

7. Verfahren nach Anspruch 4 **gekennzeichnet durch** ein Aminoalkyl-funktionelles Alkoxysilan der Formel I mit R¹ unabhängig gleich Methyl oder Ethyl und mit a = 0 und b = 0 mit c = 2 und mit der Gruppe-B gemäß Formel II mit g, e und h jeweils gleich 0 oder gemäß einer Alternative mit a = 0, b = 0, c = 3, und mit der Gruppe-B gemäß Formel II mit e = 1, d = 1, 2, 3, vorzugsweise ist d = 2 und mit g = 0, h = 0 oder mit der Gruppe-B gemäß Formel II mit e = g = 0 oder 1, und d = f = 2 oder 3 und h = 0 mit c = 2 oder mit der Gruppe-B gemäß Formel III mit j = 3 und p gleich 1 oder 2.

8. Verfahren nach Anspruch 4 **gekennzeichnet durch** ein Aminoalkyl-funktionelles Alkoxysilan der Formel I mit R¹ unabhängig gleich Methyl oder Ethyl und mit a = 0 und b = 0 mit c = 1 und mit der Gruppe-B gemäß Formel II mit g, e und h jeweils gleich 0 oder gemäß einer Alternative mit a = 0, b = 0, c = 3, und mit der Gruppe-B gemäß Formel II mit e = 1, d = 1, 2, 3, vorzugsweise ist d = 2 und mit g = 0, h = 0 oder mit der Gruppe-B gemäß Formel II mit e = g = 0 oder 1, und d = f = 2 oder 3 und h = 0 mit c = 1 oder mit der Gruppe-B gemäß Formel III mit j = 3 und p gleich 1 oder 2.

9. Verfahren nach einem der Ansprüche 4 bis 8, **dadurch gekennzeichnet, dass** das Aminoalkyl-funktionelle Silan der Formel I ausgewählt ist aus 3-Aminopropyltrimethoxysilan, 3-Aminopropyltriethoxysilan, 3-Aminopropylmethyldimethoxysilan, 3-Aminpropylmethyldiethoxysilan, 1-Aminomethyltrimethoxysilan, 1-Aminomethyltriethoxysilan, 2-Aminoethyltrimethoxysilan, 2-Aminoethyltriethoxysilan, 3-Aminoisobutyltrimethoxysilan, 3-Aminoisobutyltriethoxysilan, N-n-Butyl-3-aminopropyltriethoxysilan, N-n-Butyl-3-aminopropylmethyldiethoxysilan, N-n-Butyl-3-aminopropyltrimethoxysilan, N-n-Butyl-3-aminopropylmethyldimethoxysilan, N-n-Butyl-1-amino-methyltriethoxysilan, N-n-Butyl-1-aminomethylmethyldimethoxysilan, N-n-Butyl-1-aminomethyltrimethoxysilan, N-n-Butyl-1-aminomethylmethyltriethoxysilan, Benzyl-3-aminopropyltrimethoxysilan, Benzyl-3-aminopropyltriethoxysilan, Benzyl-2-aminoethyl-3-aminopropyltrimethoxysilan, Benzyl-2-aminoethyl-3-aminopropyltriethoxysilan, Diaminoethylen-3-propyltrimethoxysilan, Diaminoethylen-3-propyltriethoxysilan, Triaminodiethylen-3-propyltrimethoxysilan, Triaminodiethylen-3-propyltriethoxysilan, (2-Aminoethylamino)-ethyltrimethoxysilan, (2-Aminoethylamino)-ethyltriethoxysilan, (1-Aminoethylamino)-methyltrimethoxysilan und (1-Aminoethylamino)-methyltriethoxysilan, wobei insbesondere Di- und oder Triaminoalkoxysilane bevorzugt sind.

10. Verfahren nach Anspruch 4, **dadurch gekennzeichnet, dass** das Acrylsäureanhydrid der Formel IV das Methacrylsäureanhydrid oder das (unsubstituierte) Acrylsäureanhydrid ist.

11. Verfahren nach einem der Ansprüche 4 bis 10, **dadurch gekennzeichnet, dass** die definierte Menge Wasser 0,1 bis 2,0 mol Wasser pro Mol Siliciumatome der im Verfahren eingesetzten Aminoalkyl-funktionellen Siliciumverbindung beträgt, insbesondere der Formel I, vorzugweise 0,3 bis 1,5 mol Wasser pro Mol Siliciumatome der vorgenannten Siliciumverbindung, besonders bevorzugt 0,5 bis 1,0 mol Wasser pro Mol Siliciumatome der Siliciumverbindung.

12. Verfahren nach einem der Ansprüche 4 bis11, **dadurch gekennzeichnet, dass** die definierte Menge Wasser in einem Verfahrensschritt vor dem Schritt der Umsetzung mit der Komponente B eingestellt wird.

13. Verfahren nach einem der Ansprüche 4 bis 12, insbesondere zur Herstellung von im Wesentlichen wasserlöslichen Acrylamido-funktionellen Siloxanolen, indem
- in einem Schritt (I) umfassend die nachfolgenden Teilschritte die Komponente A, eine Aminoalkyl-funktionelle Siliciumverbindung, die mindestens ein Aminoalkyl-funktionellen Alkoxysilan der Formel I ist, wie in einem der Ansprüche 1, 5 bis 9 definiert, optional im Gemisch mit einem Verdünnungsmittel, vorzugsweise einem Alkohol, besonders bevorzugt Methanol, Ethanol oder Propanol,
- mit einer definierten Menge Wasser versetzt wird,
- optional wird der Hydrolysealkohol und/oder das zugesetzte Verdünnungsmittel zumindest teilweise entfernt,
- zum resultierenden Gemisch wird das Acrylsäureanhydrid der Formel IV zugesetzt, vorzugsweise wird das Acrylsäureanhydrid der Formel IV so zudosiert, dass die Temperatur des Gemisches nicht über 75 °C ansteigt,
- optional wird dem Gemisch ein Stabilisator zugesetzt,
- in einem Schritt (IIa) gemäß einer Alternative wird nach dem Schritt (I) der Hydrolysealkohol und/oder das zugesetzte Verdünnungsmittel zumindest teilweise unter Umgebungs- oder vermindertem Druck und erhöhter Temperatur entfernt, vorzugsweise wird im Schritt (IIa) Wasser zugesetzt, oder
- in einem Schritt (IIb) gemäß einer weiteren Alternative wird nach dem Schritt (I) dem Gemisch eine Base zugesetzt,
- Abtrennen des Niederschlages, optional
- Zugabe einer organischen Säure und zumindest teilweises Entfernen des Hydrolysealkohols und/oder des Verdünnungsmittels.

14. Verfahren nach einem der Ansprüche 4 bis 13, **dadurch gekennzeichnet, dass** das molare Verhältnis der Stickstoffatome der Aminoalkyl-funktionellen Siliciumverbindung, insbesondere des Aminoalkyl-funktionellen Silane der Formel I, zum molaren Verhältnis der aus dem Acrylsäureanhydrid der Formel IV freigesetzten Acrylcarbonyl-Funktion im Bereich von 1 zu 5 bis 5 zu 1, insbesondere 1 zu 2 bis 2 zu 1, bevorzugt 1 zu 1,5 bis 1,5 zu 1, besonders bevorzugt um 1 zu 1 mit einer Schwankungsbreite von plus/minus 0,5, vorzugsweise 0,2 liegt.

15. Verfahren nach einem der Ansprüche 4 bis 14, **dadurch gekennzeichnet, dass** der Wirkstoffgehalt an Acrylamido-funktionellen Siloxanolen auf 0,0001 bis 99,9 Gew.% in der Gesamtzusammensetzung eingestellt wird, insbesondere auf 10 bis 80 Gew.-%, bevorzugt auf 20 bis 60 Gew.-%, besonders bevorzugt auf 35 bis 60 Gew.-%.

16. Verfahren nach einem der Ansprüche 4 bis 15, **dadurch gekennzeichnet, dass** der pH-Wert der Zusammensetzung zwischen 5 bis 11 liegt, insbesondere zwischen 6 bis 10, und insbesondere auf einen Wert unterhalb von 8 in wässriger Phase eingestellt wird, besonders bevorzugt zwischen 3 bis 8.

17. Verfahren nach einem der Ansprüche 4 bis 16, **dadurch gekennzeichnet, dass** flüchtige Verdünnungsmittel und Hydrolysealkohol bis auf einen Gehalt in der Gesamtzusammensetzung von kleiner gleich 12 Gew.-% bis 0 Gew.-% entfernt werden, wobei das Entfernen durch Destillation unter vermindertem Druck im Bereich von 1 bis 1000 mbar bei einer Temperatur von unter 60 °C Sumpftemperatur erfolgt, vorzugsweise auf kleiner gleich 10 Gew.-%, besonders bevorzugt kleiner gleich 5 Gew.-%, ganz besonders bevorzugt kleiner gleich 2 Gew.-% bis 0,0001 Gew.-%, insbesondere kleiner gleich 1 bis 0,0001 Gew.-%, wobei das Entfernen vorzugsweise durch Destillation, insbesondere unter vermindertem Druck im Bereich von 1 bis 1000 mbar, bevorzugt von 0,001 bis 350 mbar, erfolgt.

18. Verwendung einer Zusammensetzung nach einem der Ansprüche 1 bis 3 oder der Verfahrensprodukte einer der Ansprüche 4 bis 17 als Haftvermittler, zur Funktionalisierung von Glas, insbesondere zur Funktionalisierung von Glasfasern, zur Modifizierung von Füllstoffen, Pigmenten und/oder anorganischen Oberflächen, insbesondere als Füllstoffbeschichtung, Beschichtung von Pigmenten, Beschichtung von anorganischen Oberflächen, in Zahnabformmassen, in Dentalkunststoffmassen, als Additiv in Polymeren, in Klebstoffen, in Dichtstoffen, in Faserverbundwerkstoffen, zusammen mit Polymeren, insbesondere Thermoplasten, Duroplasten, Elastomeren, zur Funktionalisierung von Polymeren, zur Einstellung der Hydrophilie von Polymeren.

## Claims

1. Composition comprising acrylamido-functional siloxanols derived from a
a) reaction of a component A which is an aminoalkyl-functionalized silicon compound selected from
(i) an aminoalkyl-functional alkoxysilane of the formula I or a mixture of aminoalkyl-functional alkoxysilanes of the formula I is reacted, each in the presence of a defined amount of water,
or
(ii) a hydrolysis or condensation product of at least one aminoalkyl-functional alkoxysilane of the formula I
or
(iii) a mixture comprising at least one aminoalkyl-functional alkoxysilane of the formula I and a hydrolysis and/or condensation product of at least one aminoalkyl-functional alkoxysilane of the formula I,
wherein the aminoalkyl-functional alkoxysilane corresponds to the formula I
(R¹O)_{3-a-b}(R²)ₐSi(B)_{1+b} (I)
and the group B in formula I independently corresponds to a group of the formula II
-(CH₂)_{c}-[(NH)(CH₂)_{d}]ₑ[(NH)](CH₂)_{f}]_{g}NH₍₂₋ₕ₎ R³ₕ (II),
in formula I with R¹ independently a linear, branched or cyclic alkyl group having 1 to 8 carbon atoms, R² independently a linear, branched or cyclic alkyl group having 1 to 8 carbon atoms, and in formula II with R³ independently a linear, branched or cyclic alkyl, aryl or alkylaryl group having 1 to 8 carbon atoms; and in formula I a is independently 0 or 1, b is independently 0 or 1, in formula II c is independently selected from 1, 2, 3, 4, 5 and 6, d is independently selected from 1, 2, 3, 4, 5 and 6, e is independently selected from 0, 1, 2, 3, 4, 5 and 6, f is independently selected from 1, 2, 3, 4, 5 and 6, g is independently selected from 0, 1, 2, 3, 4, 5 and 6, and h is independently 0 or 1, or the B group in formula I corresponds to a group of the formula III
-(CH₂)ⱼ-NH₂₋ₚ(CH₂-CH₂-NH₂)ₚ (III)
with j = 1, 2 or 3 and p = 0, 1 or 2, p preferably being selected from 0 and 1,
and subsequently is reacted with a component B which is an acrylic anhydride,
and optionally
b) removal of at least a portion of the alcohol of hydrolysis, with optional addition of further water in this step for removal of the alcohol of hydrolysis.

2. Composition according to Claim 1, **characterized in that** the
acrylic anhydride corresponds to the formula IV
(CHR⁵=CR⁴CO)₂O (IV)
where R⁴ is independently a hydrogen atom or a methyl group and R⁵ is independently a hydrogen atom or a methyl group, R⁵ preferably being a hydrogen atom.

3. Composition according to Claim 1 or 2, **characterized in that** at least one water-soluble acrylamido-functional siloxanol, in idealized form, corresponds to the general formula V
(R¹O)[(R¹O)₁₋ₐ(R²)ₐSi(C)_{1+b}O]ᵤ[(Y)Si(C)_{1+b}O]_{u'} R¹ • (HX)_{z} (V) where
- C corresponds to an acrylamido group and
- Y corresponds to OR¹ or, in crosslinked and/or three-dimensionally crosslinked structures, independently to OR¹ or O_{1/2},
- where R¹ corresponds to hydrogen and R² to a linear, branched or cyclic alkyl group having 1 to 8 carbon atoms,
- HX is an acid, where X is an inorganic or organic acid radical,
- with each a independently 0 or 1, each b independently 0 or 1, b preferably being 0, with each u independently an integer greater than or equal to 2, u' greater than or equal to 0 and z greater than or equal to 0 and (u + u') greater than or equal to 2,
- where the composition is free of diluents and releases no alcohol in the course of crosslinking, the composition especially comprising water-soluble acrylamido-functional siloxanols, C especially corresponding to an acrylamido group selected from -(CH₂)_{c}-[(NH)(CH₂)_{d}]ₑ[(NH)](CH₂)_{f}]_{g}NH₍₁₋ₕ₎R³ₕ-(CO)CR⁴=CHR⁵, -(CH₂)ⱼ-NH(CH₂-CH₂-NH)-(CO)CR⁴=CHR⁵ and -(CH2)ⱼ-NH₂₋ₚ(CH₂-CH₂-NH-(CO)CR⁴=CHR⁵)ₚ, where c, d, e, f, g, h, j, p and R³, R⁴, R⁵ are each as defined above.

4. Process for preparing a composition comprising acrylamido-functional siloxanols, by
- conducting the process in at least one step in the presence of a defined amount of water of 0.1 to 2.0 mol of water per mole of silicon atoms, and
- reacting a component A, an aminoalkyl-functional silicon compound selected from
(i) at least one aminoalkyl-functional alkoxysilane or a mixture of aminoalkyl-functional alkoxysilanes of the formula I, defined as in Claim 1, or
(ii) a hydrolysis or condensation product of at least one aminoalkyl-functional alkoxysilane of the formula I or
(iii) a mixture comprising at least one aminoalkyl-functional alkoxysilane of the formula I and a hydrolysis and/or condensation product of at least one aminoalkyl-functional alkoxysilane of the formula I,
- with a component B, an acrylic anhydride of the formula IV, defined as in Claim 2, and optionally
- at least partly removing the alcohol of hydrolysis formed in the reaction.

5. Process according to Claim 4, **characterized by** an aminoalkyl-functional alkoxysilane of the formula I with R¹ independently methyl or ethyl and with a = 0 and b = 0 with c = 1, 2 or 3 and with the group B of the formula II with g = 0 and e = 1 and h = 0, d = 1, 2, 3, preferably d = 2.

6. Process according to Claim 4, **characterized by** an aminoalkyl-functional alkoxysilane of the formula I with R¹ independently methyl or ethyl and with a = 0 and b = 0 with c = 3 and with the group B of the formula II with g, e and h each 0 or, in an alternative, with a = 0, b = 0, c = 3, and with the group B of the formula II with e = 1, d = 1, 2, 3, preferably d = 2 and with g = 0, h = 0 or with the group B of the formula II with e = g = 0 or 1, and d = f = 2 or 3 and h = 0 with c = 3 or with the group B of the formula III with j = 3 and p = 1 or 2.

7. Process according to Claim 4, **characterized by** an aminoalkyl-functional alkoxysilane of the formula I with R¹ independently methyl or ethyl and with a = 0 and b = 0 with c = 2 and with the group B of the formula II with g, e and h each 0 or, in an alternative, with a = 0, b = 0, c = 3, and with the group B of the formula II with e = 1, d = 1, 2, 3, preferably d = 2 and with g = 0, h = 0 or with the group B of the formula II with e = g = 0 or 1, and d = f = 2 or 3 and h = 0 with c = 2 or with the group B of the formula III with j = 3 and p = 1 or 2.

8. Process according to Claim 4, **characterized by** an aminoalkyl-functional alkoxysilane of the formula I with R¹ independently methyl or ethyl and with a = 0 and b = 0 with c = 1 and with the group B of the formula II with g, e and h each 0 or, in an alternative, with a = 0, b = 0, c = 3, and with the group B of the formula II with e = 1, d = 1, 2, 3, preferably d = 2 and with g = 0, h = 0 or with the group B of the formula II with e = g = 0 or 1, and d = f = 2 or 3 and h = 0 with c = 1 or with the group B of the formula III with j = 3 and p = 1 or 2.

9. Process according to any of Claims 4 to 8, **characterized in that** the aminoalkyl-functional silane of the formula I is selected from 3-aminopropyltrimethoxysilane, 3-aminopropyltriethoxysilane, 3-aminopropylmethyldimethoxysilane, 3-aminopropylmethyldiethoxysilane, 1-aminomethyltrimethoxysilane, 1-aminomethyltriethoxysilane, 2-aminoethyltrimethoxysilane, 2-aminoethyltriethoxysilane, 3-aminoisobutyltrimethoxysilane, 3-aminoisobutyltriethoxysilane, N-n-butyl-3-aminopropyltriethoxysilane, N-n-butyl-3-aminopropylmethyldiethoxysilane, N-n-butyl-3-aminopropyltrimethoxysilane, N-n-butyl-3-aminopropylmethyldimethoxysilane, N-n-butyl-1-aminomethyltriethoxysilane, N-n-butyl-1-aminomethylmethyldimethoxysilane, N-n-butyl-1-aminomethyltrimethoxysilane, N-n-butyl-1-aminomethylmethyltriethoxysilane, benzyl-3-aminopropyltrimethoxysilane, benzyl-3-aminopropyltriethoxysilane, benzyl-2-aminoethyl-3-aminopropyltrimethoxysilane, benzyl-2-aminoethyl-3-aminopropyltriethoxysilane, diaminoethylene-3-propyltrimethoxysilane, diaminoethylene-3-propyltriethoxysilane, triaminodiethylene-3-propyltrimethoxysilane, triaminodiethylene-3-propyltriethoxysilane, (2-aminoethylamino)ethyltrimethoxysilane, (2-aminoethylamino)ethyltriethoxysilane, (1-aminoethylamino)methyltrimethoxysilane and (1-aminoethylamino)methyltriethoxysilane, preference being given especially to di- and/or triaminoalkoxysilanes.

10. Process according to Claim 4, **characterized in that** the acrylic anhydride of the formula IV is methacrylic anhydride or (unsubstituted) acrylic anhydride.

11. Process according to any of Claims 4 to 10, **characterized in that** the defined amount of water is 0.1 to 2.0 mol of water per mole of silicon atoms in the aminoalkyl-functional silicon compound used in the process, especially of the formula I, preferably 0.3 to 1.5 mol of water per mole of silicon atoms in the aforementioned silicon compound, especially preferably 0.5 to 1.0 mol of water per mole of silicon atoms in the silicon compound.

12. Process according to any of Claims 4 to 11, **characterized in that** the defined amount of water is established in a process step prior to the step of reaction with component B.

13. Process according to any of Claims 4 to 12, especially for preparing essentially water-soluble acrylamido-functional siloxanols,
by
- in a step (I) comprising the component steps which follow, admixing component A, an aminoalkyl-functional silicon compound, the at least one aminoalkyl-functional alkoxysilane of the formula I as defined in any of Claims 1, 5 to 9, optionally in a mixture with a diluent, preferably an alcohol, more preferably methanol, ethanol or propanol,
- with a defined amount of water,
- optionally at least partly removing the alcohol of hydrolysis and/or the added diluent,
- adding the acrylic anhydride of the formula IV to the resulting mixture, preferably metering in the acrylic anhydride of the formula IV such that the temperature of the mixture does not rise above 75°C,
- optionally adding a stabilizer to the mixture,
- in a step (IIa), in an alternative, after step (I), at least partly removing the alcohol of hydrolysis and/or the added diluent under ambient or reduced pressure and elevated temperature, preference being given to adding water in step (IIa), or
- in a step (IIb), in a further alternative, after step (I), adding a base to the mixture,
- removing the precipitate, optionally
- adding an organic acid and at least partly removing the alcohol of hydrolysis and/or the diluent.

14. Process according to any of Claims 4 to 13, **characterized in that** the molar ratio of the nitrogen atoms in the aminoalkyl-functional silicon compound, especially in the aminoalkyl-functional silanes of the formula I, to the molar ratio of the acryloylcarbonyl function released from the acrylic anhydride of the formula IV is in the range from 1:5 to 5:1, especially 1:2 to 2:1, preferably 1:1.5 to 1.5:1, more preferably 1:1 with a range of variation of plus/minus 0.5, preferably 0.2.

15. Process according to any of Claims 4 to 14, **characterized in that** the active ingredient content of the acrylamido-functional siloxanols in the overall composition is adjusted to 0.0001 to 99.9% by weight, especially to 10 to 80% by weight, preferably to 20 to 60% by weight, more preferably to 35 to 60% by weight.

16. Process according to any of Claims 4 to 15, **characterized in that** the pH of the composition is between 5 and 11, especially between 6 and 10, and is especially adjusted to a value below 8 in the aqueous phase, more preferably between 3 and 8.

17. Process according to any of Claims 4 to 16, **characterized in that** volatile diluent and alcohol of hydrolysis are removed down to a content in the overall composition of less than or equal to 12% by weight to 0% by weight, the removal being effected by distillation under reduced pressure in the range from 1 to 1000 mbar at a temperature of below bottom temperature 60°, preferably to less than or equal to 10% by weight, more preferably less than or equal to 5% by weight, even more preferably less than or equal to 2% by weight to 0.0001% by weight, especially less than or equal to 1 to 0.0001% by weight, the removal preferably being effected by distillation, especially under reduced pressure in the range from 1 to 1000 mbar, preferably from 0.001 to 350 mbar.

18. Use of a composition according to any of Claims 1 to 3 or of the process products of any of Claims 4 to 17 as an adhesion promoter, for functionalization of glass, especially for functionalization of glass fibres, for modification of fillers, pigments and/or inorganic surfaces, especially as a filler coating, coating of pigments, coating of inorganic surfaces, in dental impression compounds, in dental polymer compounds, as an additive in polymers, in adhesives, in sealants, in fibre composite materials, together with polymers, especially thermoplastics, thermosets, elastomers, for functionalization of polymers, for adjusting the hydrophilicity of polymers.

## Revendications

1. Composition comprenant des siloxanols à fonction acrylamido, qui sont dérivés d'une
a) réaction d'un composant A, qui est un composé de silicium à fonctionnalisation aminoalkyle, choisi parmi :
(i) un alcoxysilane à fonction aminoalkyle de formule I ou un mélange d'alcoxysilanes à fonction aminoalkyle de formule I est mis en réaction, chacun en présence d'une quantité définie d'eau,
ou
(ii) un produit d'hydrolyse ou de condensation d'au moins un alcoxysilane à fonction aminoalkyle de formule I,
ou
(iii) un mélange comprenant au moins un alcoxysilane à fonction aminoalkyle de formule I et un produit d'hydrolyse et/ou de condensation d'au moins un alcoxysilane à fonction aminoalkyle de formule I, l'alcoxysilane à fonction aminoalkyle correspondant à la formule I :
(R¹O)_{3-a-b}(R²)ₐSi(B)_{1+b} (I)
et le groupe B dans la formule I correspondant indépendamment à un groupe de formule II :
-(CH₂)_{c}-[(NH)(CH₂)_{d}]ₑ[(NH)(CH₂)_{f}]_{g}NH₍₂₋ₕ₎R³ₕ (II)
avec, dans la formule I, les R¹ représentant indépendamment un groupe alkyle linéaire, ramifié ou cyclique de 1 à 8 atomes C, les R² représentant indépendamment un groupe alkyle linéaire, ramifié ou cyclique de 1 à 8 atomes C, et, dans la formule II, les R³ représentant indépendamment un groupe alkyle, aryle ou alkylaryle linéaire, ramifié ou cyclique de 1 à 8 atomes C ; et, dans la formule I, les a représentant indépendamment 0 ou 1, les b représentant indépendamment 0 ou 1, dans la formule II, les c étant choisis indépendamment parmi 1, 2, 3, 4, 5 et 6, les d étant choisis indépendamment parmi 1, 2, 3, 4, 5 et 6, les e étant choisis indépendamment parmi 0, 1, 2, 3, 4, 5 et 6, les f étant choisis indépendamment parmi 1, 2, 3, 4, 5 et 6, les g étant choisis indépendamment parmi 0, 1, 2, 3, 4, 5 et 6, et les h étant choisis indépendamment parmi 0 ou 1, ou le groupe B dans la formule I correspondant à un groupe de formule III :
-(CH₂)ⱼ-NH₂₋ₚ(CH₂-CH₂-NH₂)ₚ (III)
avec j = 1, 2 ou 3, et p = 0, 1 ou 2, p étant de préférence choisi parmi 0 et 1,
et puis est mis en réaction avec un composant B, qui est un anhydride de l'acide acrylique,
et éventuellement
b) l'élimination d'au moins une partie de l'alcool de l'hydrolyse, de l'eau supplémentaire étant éventuellement ajoutée pour l'élimination de l'alcool de l'hydrolyse lors de cette étape.

2. Composition selon la revendication 1, **caractérisée en ce que** l'anhydride de l'acide acrylique correspond à la formule IV :
(CHR⁵=CR⁴CO)₂O (IV)
dans laquelle les R⁴ représentent indépendamment un atome d'hydrogène ou un groupe méthyle, et les R⁵ représentent indépendamment un atome d'hydrogène ou un groupe méthyle, R⁵ représentant de préférence un atome d'hydrogène.

3. Composition selon la revendication 1 ou 2, **caractérisée en ce qu'**au moins un siloxanol à fonction acrylamido soluble dans l'eau correspond idéalement à la formule générale V :
(R¹O)[(R¹O)₁₋ₐ(R²)ₐSi(C)_{1+b}O]ᵤ[(Y)Si(C)_{1+b}O]_{u'} R¹•(HX)_{z} (V),
dans laquelle
- C correspond à un groupe acrylamido, et
- les Y correspondent à OR¹ ou, dans des structures réticulées et/ou réticulées dans l'espace, indépendamment les uns des autres à OR¹ ou O_{1/2},
- R¹ correspondant à l'hydrogène et R² correspondant à un groupe alkyle linéaire, ramifié ou cyclique de 1 à 8 atomes C,
- HX représente un acide, X étant un radical acide inorganique ou organique,
- avec les a représentant chacun indépendament 0 ou 1, les b représentant chacun indépendament 0 ou 1, b représentant de préférence 0, avec à chaque fois indépendamment un nombre entier u supérieur ou égal à 2, u' supérieur ou égal à 0, et z supérieur ou égal à 0 et (u+u') supérieur ou égal à 2,
- la composition étant exempte de diluants et aucun alcool supplémentaire n'étant libéré lors de la réticulation, la composition comprenant notamment des siloxanols à fonction acrylamido solubles dans l'eau, C correspondant notamment à un groupe acrylamido choisi parmi :
-(CH₂)_{c}-[(NH)(CH₂)_{d}]ₑ[(NH)(CH₂)_{f}]_{g}NH₍₁₋ₕ₎R³ₕ-(CO)CR⁴=CHR⁵,
-(CH₂)ⱼ-NH(CH₂-CH₂-NH)-(CO)CR⁴=CHR⁵ et
-(CH²)ⱼ-NH₂₋ₚ(CH₂-CH₂-NH-(CO)CR⁴=CHR⁵)ₚ, c, d, e, f, g, h, j, p, ainsi que R³, R⁴, R⁵ étant tels que définis précédemment.

4. Procédé de fabrication d'une composition comprenant des siloxanols à fonction acrylamido, selon lequel
- le procédé est réalisé lors d'au moins une étape en présence d'une quantité définie d'eau de 0,1 à 2,0 moles d'eau par mole d'atomes de silicium, et
- un composant A, un composé de silicium à fonction aminoalkyle, choisi parmi :
(i) au moins un alcoxysilane à fonction aminoalkyle ou un mélange d'alcoxysilanes à fonction aminoalkyle de formule I, telle que définie dans la revendication 1, ou
(ii) un produit d'hydrolyse ou de condensation d'au moins un alcoxysilane à fonction aminoalkyle de formule I,
ou
(iii) un mélange comprenant au moins un alcoxysilane à fonction aminoalkyle de formule I et un produit d'hydrolyse et/ou de condensation d'au moins un alcoxysilane à fonction aminoalkyle de formule I,
- est mis en réaction avec un composant B, un anhydride de l'acide acrylique de formule IV, telle que définie dans la revendication 2, et éventuellement
- l'alcool de l'hydrolyse formé lors de la réaction est au moins partiellement éliminé.

5. Procédé selon la revendication 4, **caractérisé par** un alcoxysilane à fonction aminoalkyle de formule I avec les R¹ représentant indépendamment méthyle ou éthyle, et avec a = 0 et b = 0, avec c = 1, 2 ou 3, et avec le groupe B selon la formule II avec g = 0 et e = 1 et h = 0, d = 1, 2, 3, de préférence d = 2.

6. Procédé selon la revendication 4, **caractérisé par** un alcoxysilane à fonction aminoalkyle de formule I avec les R¹ représentant indépendamment méthyle ou éthyle, et avec a = 0 et b = 0, avec c = 3, et avec le groupe B selon la formule II avec g, e et h représentant chacun 0 ou, en variante, avec a = 0, b = 0, c = 3, et avec le groupe B selon la formule II avec e = 1, d = 1, 2, 3, de préférence d = 2, et avec g = 0, h = 0, ou avec le groupe B selon la formule II avec e = g = 0 ou 1, et d = f = 2 ou 3, et h = 0 avec c = 3, ou avec le groupe B selon la formule III avec j = 3 et p = 1 ou 2.

7. Procédé selon la revendication 4, **caractérisé par** un alcoxysilane à fonction aminoalkyle de formule I avec les R¹ représentant indépendamment méthyle ou éthyle, et avec a = 0 et b = 0, avec c = 2, et avec le groupe B selon la formule II avec g, e et h représentant chacun 0 ou, en variante, avec a = 0, b = 0, c = 3, et avec le groupe B selon la formule II avec e = 1, d = 1, 2, 3, de préférence d = 2, et avec g = 0, h = 0, ou avec le groupe B selon la formule II avec e = g = 0 ou 1, et d = f = 2 ou 3, et h = 0 avec c = 2, ou avec le groupe B selon la formule III avec j = 3 et p = 1 ou 2.

8. Procédé selon la revendication 4, **caractérisé par** un alcoxysilane à fonction aminoalkyle de formule I avec les R¹ représentant indépendamment méthyle ou éthyle, et avec a = 0 et b = 0, avec c = 1, et avec le groupe B selon la formule II avec g, e et h représentant chacun 0 ou, en variante, avec a = 0, b = 0, c = 3, et avec le groupe B selon la formule II avec e = 1, d = 1, 2, 3, de préférence d = 2, et avec g = 0, h = 0, ou avec le groupe B selon la formule II avec e = g = 0 ou 1, et d = f = 2 ou 3, et h = 0 avec c = 1, ou avec le groupe B selon la formule III avec j = 3 et p = 1 ou 2.

9. Procédé selon l'une quelconque des revendications 4 à 8, **caractérisé en ce que** le silane à fonction aminoalkyle de formule I est choisi parmi le 3-aminopropyltriméthoxysilane, le 3-aminopropyltriéthoxysilane, le 3-aminopropylméthyldiméthoxysilane, le 3-aminopropylméthyldiéthoxysilane, le 1-aminométhyltriméthoxysilane, le 1-aminométhyltriéthoxysilane, le 2-aminoéthyltriméthoxysilane, le 2-aminoéthyltriéthoxysilane, le 3-aminoisobutyltriméthoxysilane, le 3-aminoisobutyltriéthoxysilane, le N-n-butyl-3-aminopropyltriéthoxysilane, le N-n-butyl-3-aminopropylméthyldiéthoxysilane, le N-n-butyl-3-aminopropyltriméthoxysilane, le N-n-butyl-3-aminopropylméthyldiméthoxysilane, le N-n-butyl-1-amino-méthyltriéthoxysilane, le N-n-butyl-1-aminométhylméthyldiméthoxysilane, le N-n-butyl-1-aminométhyltriméthoxysilane, le N-n-butyl-1-aminométhylméthyltriéthoxysilane, le benzyl-3-aminopropyltriméthoxysilane, le benzyl-3-aminopropyltriéthoxysilane, le benzyl-2-aminoéthyl-3-aminopropyltriméthoxysilane, le benzyl-2-aminoéthyl-3-aminopropyltriéthoxysilane, le diaminoéthylène-3-propyltriméthoxysilane, le diaminoéthylène-3-propyltriéthoxysilane, le triaminodiéthylène-3-propyltriméthoxysilane, le triaminodiéthylène-3-propyltriéthoxysilane, le (2-aminoéthylamino)-éthyltriméthoxysilane, le (2-aminoéthylamino)-éthyltriéthoxysilane, le (1-aminoéthylamino)-méthyltriméthoxysilane et le (1-aminoéthylamino)-méthyltriéthoxysilane, les di- et/ou triaminoalcoxysilanes étant particulièrement préférés.

10. Procédé selon la revendication 4, **caractérisé en ce que** l'anhydride de l'acide acrylique de formule IV est l'anhydride de l'acide méthacrylique ou l'anhydride de l'acide acrylique (non substitué).

11. Procédé selon l'une quelconque des revendications 4 à 10, **caractérisé en ce que** la quantité définie d'eau est de 0,1 à 2,0 moles d'eau par mole d'atomes de silicium du composé de silicium à fonction aminoalkyle utilisé dans le procédé, notamment de formule I, de préférence de 0,3 à 1,5 mole d'eau par mole d'atomes de silicium du composé de silicium susmentionné, de manière particulièrement préférée de 0,5 à 1,0 mole d'eau par mole d'atomes de silicium du composé de silicium.

12. Procédé selon l'une quelconque des revendications 4 à 11, **caractérisé en ce que** la quantité définie d'eau est ajustée lors d'une étape de procédé avant l'étape de réaction avec le composant B.

13. Procédé selon l'une quelconque des revendications 4 à 12, notamment pour la fabrication de siloxanols à fonction acrylamido essentiellement solubles dans l'eau, selon lequel
- lors d'une étape (I) comprenant les étapes partielles suivantes, le composant A, un composé de silicium à fonction aminoalkyle, qui est au moins un alcoxysilane à fonction aminoalkyle de formule I, telle que définie dans l'une quelconque des revendications 1, 5 à 9, éventuellement en mélange avec un diluant, de préférence un alcool, de manière particulièrement préférée le méthanol, l'éthanol ou le propanol,
- est mélangé avec une quantité définie d'eau,
- l'alcool de l'hydrolyse et/ou le diluant ajouté sont éventuellement au moins partiellement éliminés,
- l'anhydride de l'acide acrylique de formule IV est ajouté au mélange résultant, l'anhydride de l'acide acrylique de la formule IV étant de préférence ajouté de telle sorte que la température du mélange n'augmente pas au-dessus de 75 °C,
- un stabilisateur est éventuellement ajouté au mélange,
- lors d'une étape (IIa), selon une variante, après l'étape (I), l'alcool de l'hydrolyse et/ou le diluant ajouté sont au moins partiellement éliminés sous pression ambiante ou réduite et température élevée, de l'eau étant de préférence ajoutée à l'étape (IIa), ou
- lors d'une étape (IIb), selon une autre variante, après l'étape (I), une base est ajoutée au mélange,
- le précipité est séparé, éventuellement
- un acide organique est ajouté et l'alcool de l'hydrolyse et/ou le diluant sont au moins partiellement éliminés.

14. Procédé selon l'une quelconque des revendications 4 à 13, **caractérisé en ce que** le rapport molaire entre les atomes d'azote du composé de silicium à fonction aminoalkyle, notamment du silane à fonction aminoalkyle de formule I, et la fonction acrylcarbonyle libérée par l'anhydride de l'acide acrylique de formule IV se situe dans la plage allant de 1 sur 5 à 5 sur 1, notamment de 1 sur 2 à 2 sur 1, de préférence de 1 sur 1,5 à 1,5 sur 1, de manière particulièrement préférée aux alentours de 1 sur 1, avec une marge de fluctuation de plus/moins 0,5, de préférence 0,2.

15. Procédé selon l'une quelconque des revendications 4 à 14, **caractérisé en ce que** la teneur en agent actif siloxanols à fonction acrylamido est ajustée de 0,0001 à 99,9 % en poids dans la composition totale, notamment de 10 à 80 % en poids, de préférence de 20 à 60 % en poids, de manière particulièrement préférée de 35 à 60 % en poids.

16. Procédé selon l'une quelconque des revendications 4 à 15, **caractérisé en ce que** le pH de la composition est compris entre 5 et 11, notamment entre 6 et 10, et est notamment ajusté à une valeur inférieure à 8 dans la phase aqueuse, de manière particulièrement préférée entre 3 et 8.

17. Procédé selon l'une quelconque des revendications 4 à 16, **caractérisé en ce que** les diluants liquides et l'alcool de l'hydrolyse sont éliminés jusqu'à une teneur dans la composition totale de 12 % en poids ou moins à 0 % en poids, l'élimination ayant lieu par distillation sous pression réduite dans la plage allant de 1 à 1 000 mbar à une température inférieure à une température de fond de 60 °C, de préférence à 10 % en poids ou moins, de manière particulièrement préférée à 5 % en poids ou moins, de manière tout particulièrement préférée à 2 % en poids ou moins à 0,0001 % en poids, notamment 1 ou moins à 0,0001 % en poids, l'élimination ayant de préférence lieu par distillation, notamment sous pression réduite dans la plage allant de 1 à 1 000 mbar, de préférence de 0,001 à 350 mbar.

18. Utilisation d'une composition selon l'une quelconque des revendications 1 à 3 ou des produits de procédé selon l'une quelconque des revendications 4 à 17 en tant que promoteur d'adhésion, pour la fonctionnalisation de verre, notamment pour la fonctionnalisation de fibres de verre, pour la modification de charges, de pigments et/ou de surfaces inorganiques, notamment en tant que revêtement de charges, revêtement de pigments, revêtement de surfaces inorganiques, dans des matériaux de moulage dentaires, dans des matières plastiques dentaires, en tant qu'additif dans des polymères, dans des adhésifs, dans des agents d'étanchéité, dans des matériaux composites fibreux, conjointement avec des polymères, notamment des thermoplastiques, des duroplastiques, des élastomères, pour la fonctionnalisation de polymères, pour l'ajustement de l'hydrophilie de polymères.
